# EUROPEAN PATENT SPECIFICATION

(11) **EP 2 255 015 B1**
(45) Date of publication and mention of the grant of the patent: **29.04.2015**
(21) Application number: 09711667.7
(22) Date of filing: 18.02.2009
(51) Int. Cl.: C12Q 1/68

(54) **METHODS OF PRODUCING HOMOGENEOUS PLASTIC-ADHERENT APTAMER-MAGNETIC BEAD-FLUOROPHORE SANDWICH ASSAYS**
VERFAHREN ZUR HERSTELLUNG HOMOGENER APTAMER-MAGNETKÜGELCHEN-FLUOROPHOR-SANDWICH-TESTS MIT KUNSTSTOFFHAFTUNG
PROCÉDÉS DE PRODUCTION DE DOSAGES SANDWICH BASÉS SUR UN CONJUGUÉ FLUOROPHORE-BILLE MAGNÉTIQUE-APTAMÈRE ADHÉRENT AU PLASTIQUE

(30) Priority: 21.02.2008 US 66506 P; 16.06.2008 US 132147 P
(43) Date of publication of application: 01.12.2010
(73) Proprietor: OTC Biotechnologies, Llc, San Antonio, TX 78229 (US)
(72) Inventor: BRUNO, John, G., San Antonio, TX 78245 (US)
(74) Representative: Colens, Alain M.G.M.
(86) International application number: PCT/IB2009/000290
(87) International publication number: WO 2009/104075

(56) References cited:
- WO-A2-2007/092941
- US-A- 5 376 313
- US-A1- 2006 014 172
- US-A1- 2006 257 915
- US-A1- 2006 257 958
- US-A1- 2006 257 958
- US-B2- 7 198 900
- YU H: "Comparative studies of magnetic particle-based solid phase fluorogenic and electrochemiluminescent immunoassay", JOURNAL OF IMMUNOLOGICAL METHODS, ELSEVIER SCIENCE PUBLISHERS B.V.,AMSTERDAM, NL, vol. 218, no. 1-2, 1 September 1998 (1998-09-01), pages 1-8, XP004146527, ISSN: 0022-1759, DOI: DOI:10.1016/S0022-1759(98)00047-7
- TANSIL N C ET AL: "Nanoparticles in biomolecular detection", NANO TODAY, ELSEVIER, AMSTERDAM, NL, vol. 1, no. 1, 1 February 2006 (2006-02-01), pages 28-37, XP024992793, ISSN: 1748-0132, DOI: DOI:10.1016/S1748-0132(06)70020-2 [retrieved on 2006-02-01]
- SU X-L ET AL: "Quantum dot biolabeling coupled with immunomagnetic separation for detection of Escherichia coli O1517:H7", ANALYTICAL CHEMISTRY, AMERICAN CHEMICAL SOCIETY, US, vol. 76, no. 16, 15 August 2004 (2004-08-15), pages 4806-4810, XP002579713, ISSN: 0003-2700, DOI: DOI:10.1021/AC049442+ [retrieved on 2004-07-10]

## Description

### BACKGROUND OF THE INVENTION

### Field of the Invention

The present invention relates to the field of aptamer- and nucleic acid-based diagnostics. More particularly, it relates to methods for the production and use of self-assembling DNA aptamer-magnetic bead ("MB") conjugate combined with aptamer-quantum dot ("QD") or other aptamer-fluorophore conjugate sandwich assays that naturally adhere to glass and certain plastics such as polystyrene (or derivatives thereof) to enable one-step (homogeneous) tests without a wash step even after an external magnetic field is removed. Conjugation of aptamers to the MBs or QDs and other fluorophores may be accomplished by simple chemical coupling reactions through bifunctional linkers, or key functional groups such as aldehydes, carbodiimides, carboxyls, N-hydroxy-succinimide ("NHS") esters, N-oxy-succinimide ("NOS") esters, thiols, etc. or via biotin-avidin, histidine-Nickel, or other high affinity linkage systems. This one-step, washless assay format has numerous applications for sensitive detection of foodborne pathogens on and in meats, poultry, serous fluids, dairy products, fruits, vegetables, and other food matrices. The assay is also applicable to environmental analyses in soil or muddy water samples and clinical and veterinary diagnostics performed directly on whole blood, urine, saliva or other body fluids with or without sample dilution, but without a wash step. The typical wash step involves purification by removal of unwanted materials contributing to background fluorescence.

### Background Information

The most desirable of all diagnostic assay strategies are rapid one-step "bind and detect" or "homogeneous" assays that do not require a wash step and yet do not sacrifice a significant degree of sensitivity. Examples of successful one-step assay strategies include fluorescence polarization ("FP") and fluorescence resonance energy transfer ("FRET")-based assays. While both of these formats are popular, they tend to sacrifice sensitivity for speed in obtaining test results. Therefore, FP and FRET assays are typically relegated to clinical diagnostics for certain analytes that exist in relatively high concentrations (micro to milliMolar ranges) in blood, urine, or other body fluids. For analytes that exist in much lower concentrations, multi-step assays such as enzyme-linked immunosorbent assays ("ELISA"), radioimmunoassays ("RIA") and other sandwich-formatted assays such as immunomagnetic-electrochemiluminescence ("IM-ECL") assays are required to detect nanogram, nanoMolar or lower amounts of various target analytes. Typically, these types of sandwich assays will require one or more wash steps, thereby slowing their execution speed. Wash steps will be known to one skilled in the art as a generally necessary step to remove unwanted materials (besides the detected target analyte) to prevent high background fluorescence signals. Eliminating the need for a wash step is found to be desirable in the present invention because it can enhance the speed and accuracy of many assays.

DNA is well known to adhere to some glass surfaces especially if the surface is charged by rubbing. This principle is used in the electrostatic collection of genomic DNA from cell lysates which is known as "spooling" of DNA with a charged glass rod. Similarly, Allemand et al., Bensimon et al., Buck and Andrews, Dudley et al., Klein et al., Labit et al., Michalet et al., Moscoso et al., and Torres et al. teach adherence of DNA, bacteria, biofilms, and other materials to polystyrene by electrostatic and hydrophobic or other weak forces. However, Allemand et al., Bensimon et al., and Klein et al. emphasize that DNA is far more likely to bind to polystyrene and other plastics at its free 3' or 5' ends than in the mid-regions and that such binding is not instantaneous (requires one or more minutes of residence time for DNA to bind to plastic) and is pH-dependent with optimal pH for binding being acidic (at an approximate pH of 5.5, which is below the range of most biological assays). Bensimon et al. (1994) have even suggested that DNA may couple covalently to polystyrene by electrophilic addition of 5' or 3' phosphate ends (in their phosphoric acid forms) to the pi double bonds of the styrene rings or free unpolymerized alkene ends of polystyrene fibers. Such covalent bonding of DNA aptamers to polystyrene would explain the very stable and long-lasting adherence of assay materials observed and reported herein and by Bruno et al. (2008) for their *Campylobacter* assay.

While some species of bacteria can bind to plastics and glass, not all species can form such adherent biofilms. In the presently described assays, attachment of the assay components (DNA aptamers, MBs and QDs) have occurred in the presence and absence of target bacteria. Conversely, immunomagnetic ("antibody-MB") sandwich assays do not adhere to polystyrene very well at neutral or acidic pH, presumably because protein antibodies do not adhere well to plastic or glass materials at neutral or acidic pH. Proteins such as antibodies are well known to adhere to polystyrene microtiter plate wells at alkaline pH values as in the popular ELISA test formats. However, the pH for adherence of antibodies and proteins in ELISA assays is typically 8.0-9.5 and clearly not acidic as in the presently described DNA-adherent assays. Therefore, the DNA aptamer is considered to be the key component which enables adherence to polystyrene or glass or derivatives thereof and thus enables one-step washless assays. While some species of bacteria may contribute to overall adherence to the inner face of a cuvette, the DNA aptamer component appears sufficient to enable adherence of the aforementioned assays in the magnetized region because assay components (aptamer-MB conjugates) will adhere to plastic and glass even in the absence of captured bacterial cells.

### Related Literature

Allemand J.F., et al. pH-dependent specific binding and combing of DNA. Biophys. J. 73:2064-2070, 1997.

Bensimon A., et al. Alignment and sensitive detection of DNA by a moving interface. Science. 265:2096-2098, 1994.

Bensimon D., et al. Stretching DNA with a receding meniscus: experiments and models. Phys. Rev. Lett. 74:4754-4757, 1995.

Bruno J.G., Phillips T., Carrillo M.P., Crowell R. Plastic-adherent DNA aptamer-magnetic bead and quantum dot sandwich assay for Campylobacter detection. J. Fluorescence. In Press, 2008.

Bruno J.G., Carrillo M.P., Phillips T., Crowell R. Initial development of competitive FRET-aptamer assays for monitoring bone metabolism. J. Clin. Ligand Assay. In Press, 2008.

Bruno J.G., Carrillo M.P., Crowell R. Preliminary development of DNA aptamer-Fc conjugate opsonins. J. Biomedical Materials Research-Part A, In Press, 2008.

Bruno J.G., Carrillo M.P., Phillips T. In vitro antibacterial effects of anti-lipopolysaccharide DNA aptamer-Clqrs complexes. Folia Microbiologica. 53:295-302, 2008.

Bruno J.G., Carrillo M.P., Phillips T., King B. Development of DNA aptamers for cytochemical detection of acetylcholine. In Vitro Cell. Develop. Biol. - Animal. 44:63-72, 2008.

Bruno J.G., Carrillo M.P., Phillips T. Development of DNA aptamers to a Foot-and-Mouth Disease peptide for competitive FRET-based detection. J. Biomolecular Techniques. 19:109-115, 2008.

Bruno J.G., Carrillo M.P., Phillips T. Effects of immobilization chemistry on enzyme-linked aptamer assays for Leishmania surface antigens. J. Clinical Ligand Assay. 30:37-43, 2007.

Bruno J.G., Francis K., Ikanovic, M., et al. Reovirus detection using immunomagnetic-fluorescent nanoparticle sandwich assays. J. Bionanoscience. 1:84-89, 2007.

Buck J.W. and Andrews J.H. Localized, positive charge mediates adhesion of Rhodosporidium torulides to barley leaves and polystyrene. Appl. Environ. Microbiol. 65:2179-2183, 1999.

Dudley E.G., et al. An IncI1 plasmid contributes to the adherence of the atypical enteroaggregative Escherichia coli strain C1096 to cultured cells and abiotic surfaces. Infect. Immun. 74:2102-2114,2006.

Dwarakanath S., Satyanarayana S., Bruno J.G., et al. Ultra sensitive fluorescent nanoparticle-based binding assays for foodborne and waterborne pathogens of clinical interest. J. Clinical Ligand Assay. 29:136-142, 2006.

Ikanovic M., Rudzinski W.E., Bruno J.G., Dwarakanath S., et al. Fluorescence assay based on aptamer-quantum dot binding to Bacillus thuringiensis spores. J. Fluorescence. 17:193-199, 2007.

Joshi S. et al. Selection, characterization, and application of DNA aptamers for the capture and detection of Salmonella enterica serovars. Molec. Cell Probes. In Press, 2008.

Klein D.C.G., et al. Ordered stretching of single molecules of deoxyribose nucleic acid between microfabricated polystyrene lines. Appl. Phys. Lett. 78:2396-2398, 2001.

Labit H., et al. A simple and optimized method of producing silanized surfaces for FISH and replication mapping on combed DNA fibers. BioTechniques. 45:649-658, 2008.

Michalet X., et al. Dynamic molecular combing: stretching the whole human genome for high-resolution studies. Science. 277:1518-1523, 1997.

Moscoso M. et al. Biofilm formation by Streptococcus pneumoniae: Role of choline extracellular DNA, and capsular polysaccharide in microbial accretion. J. Bacteriol. 188:7785-7795, 2006.

Quast B. A compact, handheld laboratory fluorometer. American Biotechnol Lab 18:68, 2001.

Torres A.G., et al. Differential binding of Escherichia coli 0157:H7 to alfalfa, human epithelial cells, and plastic is mediated by a variety of surface structures. Appl. Environ. Microbiol. 71:8008-8015, 2005.

US Patent application 2006/0257958 discloses a device comprising a plastic cartridge containing several microchannels and a movable magnet. The device is used by binding a magnetic bead to a target molecule and then using a magnet to move the target molecule through the microchannels. Binding is detected through a detection window by determining fluorescence of the fluorophore associated to the complex. The fluorescent particles are magnetically immobilized on the opposite side of the detection window so that fluorescence could greatly suffer from interference caused by the solution between the window and the immobilized particles.

### SUMMARY OF THE INVENTION

The present invention is directed to a method as disclosed in attached claim 1.

Indeed, herein is described a new type of aptamer-MB-aptamer-QD sandwich assay and its derivative formats with variations in the fluorophore component that can be accomplished in one-step, obviating a wash step, by collecting the MBs with a strong external magnetic field onto a glass, polystyrene, other plastic or coated surface such as the inner face of a cuvette. Collection of the MBs and all attached assay components, including DNA aptamers, MBs, fluorophores and the captured analytes, into a small area on the plastic surface thereby focuses fluorescence intensity of the assay due to capture of the analyte in a thin planar area of adherence. Thus, when the adherent material is illuminated even in nearly opaque matrices such as foods or blood, the fluorescence can be detected with ultra sensitivity over background autofluorescence from the bulk solution due to partitioning and concentrating of the assay materials and captured analytes to the area of adherence. Fluorescence from uncaptured aptamer-QD or aptamer-fluorophore conjugates in the bulk solution contributes to background fluorescence, but its contribution to the total fluorescence signal is greatly minimized because it is not concentrated to the area of assay adherence. Any aptamer-QD or aptamer-fluorophore conjugates that do not bind the analyte and aptamer-MB conjugates will not be pulled toward the plastic surface nor adhere to the surface significantly and will not contribute significantly to the detection signal, but will contribute to the much weaker background fluorescence "noise" in the bulk solution. The combination of high aptamer affinity, the MBs ability to be concentrated in a defined area, and the long Stoke's shift of red-emitting QDs (i.e., high energy ultraviolet excitation with emission in the red region of the spectrum above 600 nm) contribute to the ultra sensitive nature of this one-step washless assay format. However, adherence of the assay materials and captured analytes to a small area on a clear plastic or glass surface even when the external magnetic field is removed is the key factor that enables one-step washless detection.

The present invention provides for the assembly of DNA and RNA aptamer-MB conjugates for capture of target analytes with aptamer-QD or other aptamer-fluorophore conjugates. The target analytes are molecules that it is desirable to detect such as, pathogenic bacteria, viruses, parasites, leukocytes, cancer cells, proteins, other macromolecules, toxins, pollutants, drugs, explosives, proteins, viral capsid proteins, viral polymerases, biotoxins such as bacterial toxin, botulinum, cholera, tetanus, staphylococcal enterotoxin, shigatoxins or verotoxins, algal toxins, such as brevetoxin, ciguatoxin, cyanotoxin, or saxitoxin, snake or spider venoms, clinically relevant proteins or portions of proteins (peptides) such as bone marker (e.g., collagen breakdown peptides such as CTx, NTx, OCF, Cathepsin K or its precursor ProCathepsin K, deoxypyridinoline, pyridinoline, lysyl pyridinoline, or hydroxylysyl pyridinoline) cytokines and interleukins, markers of myocardial infarctions (troponin, myoglobin, etc.), kidney disease, antibodies, autoimmune disorders, arthritis, or other clinically relevant macromolecules such as lipopolysaccharides (LPS, endotoxins), and other small molecules (where "small molecules" are defined as being those that are less than 1,000 Daltons) such as with at least two distinct epitopes from a group including the following: pesticides, natural and synthetic amino acids and their derivatives, hydroxylysine, hydroxyproline, histidine, histamine, homocysteine, DOPA, melatonin, nitrotyrosine, short chain proteolysis products, cadaverine, putrescine, polyamines, spermine, spermidine, deoxypyridinoline, pyridinoline, lysyl pyridinoline, or hydroxylysyl pyridinoline, nitrogen bases of DNA or RNA, nucleosides, nucleotides, nucleotide cyclical isoforms, cAMP, cGMP, cellular metabolites, urea, uric acid, pharmaceuticals, therapeutic drugs, vitamins, illegal drugs, narcotics, hallucinogens, gamma-hydroxybutyrate (GHB), cellular mediators, cytokines, chemokines, immune modulators, neural modulators, neurotransmitters such as acetylcholine, inflammatory modulators, prostaglandins, prostaglandin metabolites, nitoaromatic and nitramine explosives, explosive breakdown products (e.g., DNT) or byproducts, quorum sensing molecules such as AHLs, steroids, hormones, and their derivatives.

A fluorophore is a fluorescent component, or functional group, bound to a molecule. A fluorophore can be a dye, a glowing bead, a glowing liposome, a quantum dot ("QD"), a fluorescent or phosphorescent nanoparticle ("NP"), a fluorescent latex particle or microbead, a fluorescent dye molecule, such as fluorescein, carboxyfluorescein and other fluorescein derivatives, rhodamine, and their derivatives, a fluorescence resonance energy transfer ("FRET") complex such as an intrachain or competitive FRET-aptamer, or any other glowing entity capable of forming a covalent bond with the aptamer. As used herein, "other aptamer-fluorophore conjugates" includes those aptamers having a fluorophore bonded to them, such as, in addition to those listed otherwise herein, aptamer-fluorescent dye conjugates, aptamer-fluorescent microbead conjugates, or aptamer-liposome conjugates containing fluorescent dyes. In the present invention, the fluorophore acts to "report" detection of the target analytes in one rapid and washless step. The only requirement of the target is that it contains two accessible epitopes of the same or different composition and conformation to enable a sandwich assay with capture and reporter aptamer components.

The present invention utilizes a one-step assay format, which can be used for sandwich assay to detect and quantify said target analyte in said bulk solution, as well as fluorescence intensity, time-resolved fluorescence, chemiluminescence, electrical detection, electrochemical detection, electrochemiluminescence, phosphorescence, or radioisotopic detection. The one-step nature of the assay stems from the fact that the assay components capture the analyte and then stick or adhere to the inner surface of the assay substrate, generally expected to be a polystyrene plastic, glass, or other type of cuvette that is transparent or translucent enough so as to allow fluorescent light propagation, in a highly magnetized region for a brief time (5-10 minutes).

More specifically, the one-step nature of the assay stems from the ability, after the application of an external magnetic field, to magnetically separate or partition the assay materials (aptamer-MBs and aptamer-QDs or other aptamer-fluorophore conjugates) from the bulk solution and allow these materials to bind or adhere to a surface such as the inner face of a polystyrene or glass cuvette via the attractive or covalent forces between DNA and some plastics or glass, thereby increasing the signal-to-noise ratio at the surface where the magnet was placed even after the magnet or magnetic field is removed to enable fluorescence detection. The sticking of brightly fluorescing analytes to the inner plane of the cuvette leads to the ability to discriminate the sample's more intense fluorescence from background or target fluorescence from bulk solution or "signal from noise" and to make one-step homogeneous assays possible. Adherence of assay materials to the cuvette constitutes a technique that even allows for detection in dense food samples (e.g. milk, chicken and beef juice, and egg yolk samples).

A typical one-step aptamer-magnetic bead plus aptamer-quantum dot cuvette assay or test will consist of the following two components synthesized and added in any order: 1) One-hundred µg of 5'-amino modified aptamer DNA specific for one epitope on the target analyte plus 10 mM BS³ [Bis(sulfosuccinimidyl) suberate] or other appropriate amine-reactive bifunctional linker such as EDC [1-Ethyl-3-(3-dimethylaminopropyl)carbodiimide hydrochloride], Sulfo-EGS [Ethylene glycol bis(sulfosuccinimidylsuccinate)], Sulfo-SMCC [Sulfosuccinimidyl 4-[N-maleimidomethyl]-cyclohexane-1-carboxylate], glutaraldehyde, etc. plus 8 µM Qdot 655 ITK reagent (Invitrogen Corp.). These components are mixed in a 1 ml volume of 1X binding buffer ("1XBB"; 0.5 M NaCl, 10 mM Tris-HCl, 1 mM MgCl₂, pH 7.5-7.6) for 30 min at room temperature ("RT"). This aptamer-QD component is purified through Sephadex G-25 or another suitable size exclusion chromatography matrix. 2) One-hundred µg of a second 5'-amine-modified DNA aptamer with specificity to a second epitope on the target analyte plus 10 µl of tosyl-activated MBs (approximately 1 X 10⁶ MBs, 1 to 5 microns in diameter). This aptamer-MB component is incubated at 37°C for 2 or more hours and then collected with a strong magnet and washed 3 times in 1XBB. These two major components (aptamer-QD and aptamer-MB conjugates) are added to a polystyrene or other plastic cuvette with the addition of 1XBB up to a total volume of 2 ml. The cuvette is then lyophilized, back flushed with nitrogen gas and capped for long-term storage.

The invention has been described above in a typical embodiment and amounts of the assay components for food safety testing for low numbers of pathogenic bacteria. However, broad ranges of detection are required for other types of analytes. Therefore, considering aptamer affinity ranges and ranges of detectable fluorescence, the one-step cuvette assays may be described based on the following ratios of ranges for the two major assay components:
1) Aptamer-QD reagents: 0.5 - 50 nanoMoles of 5'-amono-DNA aptamer (or 10 - 1,000 µg of 60-100 base DNA in general) plus 10 - 20 miliMoles of bifunctional linker (BS³ etc., linkers are in excess) plus 0.8 - 80 µMoles of QDs.
2) Aptamer-MB reagents: 0.5 - 50 nanoMoles of 5'-amino-DNA per 10⁵ - 10⁷ tosyl-MBs or other appropriately derived MBs for DNA conjugation.

In general, affinities for antibodies and aptamers, 10-fold ranges for each assay component (i.e., 10-fold lower and higher) are anticipated by the current invention. The amounts of the assay components are intended to be varied, because the present invention envisions assays of varying sensitivity. Thus, the same basic assay can have assay component amounts modified to allow for situations wherein extreme sensitivity is required, and others situations wherein less sensitivity is acceptable for the application.

Prior to use, the one-step cuvette assay is reconstituted with a bulk solution which is to be tested for the presence of the desired target analyte. The bulk solution, which is in an amount anticipated to be approximately 2 ml, can be any number of various sample fluid matrices possibly containing target analytes including, but not limited to: natural waters, buffer, or diluted or undiluted food samples (e.g., milk, yogurt, cheeses prior to solidification, meat juices, fruit juices, eggs, rinse waters from fruit and vegetable surfaces, diluted peanut butter, etc.), diluted whole blood, serum, urine, sputum or other body fluid samples.

Along with the bulk solution, an aptamer-magnetic bead conjugate ("aptamer-MB"), and an aptamer-fluorophore conjugate are added, or can be lyophilized together in situ (in a cuvette) prior to adding the target analyte. The aptamer conjugates are chosen based upon the aptamer-MB being able to bind with the target analyte at a first binding site on the target analyte, and the aptamer-fluorophore conjugate being able to bind with the target analyte at a second binding site on the target analyte. Thus, if the target analyte is present in the bulk solution, both the aptamer-MB and an aptamer-fluorophore conjugates bind with the target analyte to form an analyte-aptamer-fluorophore complex. It is also necessary that the aptamer-MBs will not bind, base pair, or hybridize with the aptamer-fluorophores in the bulk solution. If they were to attach to each other in some way, in competition with the target analyte, then the assay would produce false positives because the MB would pull the aptamer-MB-fluorophore (without a target analyte) over to the cuvette translucent surface area to be assayed.

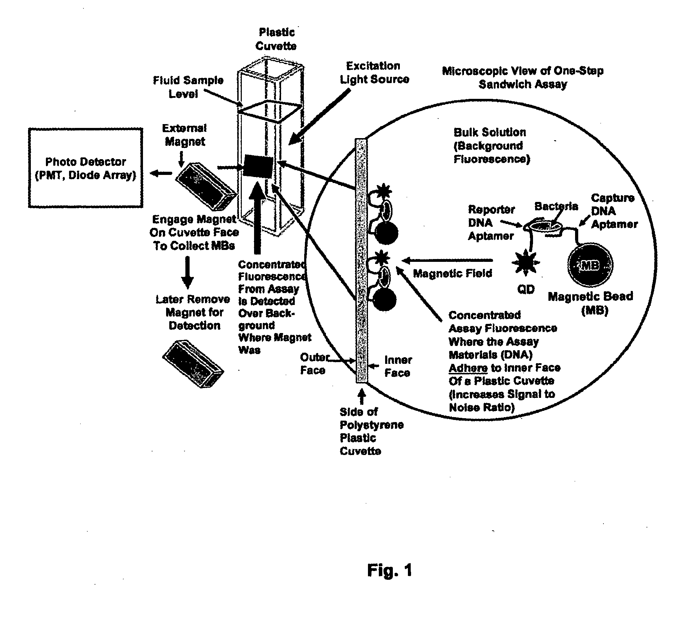

The cuvette is recapped, shaken and mixed periodically over a 15-20 minute period, allowing the aptamer-MBs to bind with target analytes at the first binding site and the aptamer-fluorophore conjugates to bind with the target analyte at the second binding site to form an analyte-aptamer-fluorophore complex. Then the cuvette is added to a rack or other device with an external magnet set at the appropriate height to cause the analyte-aptamer-fluorophore complexes to adhere to the cuvette translucent surface area by applying an external magnetic field to attract the magnetic beads. Attracted by the magnetic field, the magnetic bead pulls the remainder of the analyte-aptamer-fluorophore complex which collects any captured analytes in a band (rectangular or square) or circular pattern at the level of a fluorometer's light path. The MBs with captured assay and target analytes are collected for 5 or more minutes and then the external magnet is removed, leaving adherent fluorescent MBs, assay and target analyte components adhering on the inner surface of the plastic cuvette as shown in Figure 1. It is this partitioning and concentrating of the assay components and captured analytes to a thin adherent film on the inner face of the cuvette which enables discrimination of the intense assay fluorescence from the much weaker fluorescence of the bulk solution behind the adherent material. Thus, the analyte-aptamer-fluorophore complexes are effectively partitioned away from the remaining bulk solution to enhance detectability. This partitioning provides for a one-step, homogenous assay with high signal-to-noise ratio when the adherent assay is placed in a fluorometer and quantified with the appropriate excitation and emission wavelengths.

Although described above as a cuvette, the present invention is effective in any number of container or vessel geometries. Thus, the method of the present invention may be run in a tube, vial, dish, flow cell, cassette, cartridge, microfluidic chip, and any other similar type of containers. And, the container can be composed of a plethora of materials, in any shape and of any type as long as a planar area of assay material attachment in a viewing "window" is provided and nucleic acid aptamers can adhere to the material. Therefore, the assay format may also be applied to a flattened plastic or glass cassette or cartridge in which assay components might be magnetically pulled along a channel or path by an external magnet. Upon reaching a clear plastic or glass detection window the assay components would be allowed to reside in the detection window where they could adhere to the window's surface and be concentrated away from the bulk solution by the external magnet. Hence, several embodiments or geometries for the assay vessel are envisioned so long as the cuvette has a translucent surface area so as to enable a fluorescent assay. For example, the cuvette translucent surface area, on which said analyte-aptamer-fluorophore complex adheres, may be formed as a square, rectangular, round, oval, or flat container, vial, tube, cylinder, cassette, or cartridge.

It is anticipated that the cuvette may be made from polystyrene, clear plastic, or glass. But in addition, the chemistry of DNA attachment to the glass or plastic is not restricted to natural glass or simple polystyrene. Rather, logical derivative plastics and coatings (e.g., silanes, etc.) that include alkenes for electrophilic addition of DNA and hydrophobic coatings that may encourage weak force (van der Waals or dipole-dipole) interactions and adherence of DNA to the coated glass or plastic are also envisioned.

### BRIEF DESCRIPTION OF THE DRAWINGS

Figure 1. is a schematic illustration of how the one-step adherent sandwich assay forms and is drawn to the inner face of a plastic or glass cuvette by an external magnet.
Figure 2. shows line graphs plotting relative fluorescence intensity against the concentration of *Campylobacter jejuni (C. jejuni)* bacteria.
Figure 3. shows a series of fluorescence emission spectra related to detection of serial ten-fold dilutions of *Campylobacter jejuni* bacteria in neat buffer (1XBB) and various diluted food matrices as indicated in the figure. Excitation was at 380 nm with a photomultiplier tube setting of 900 Volts.
Figure 4 illustrates a typical one-step assay capable of detecting 10 live *C. jejuni* bacteria in undiluted chicken "juice" (serous fluid collected from chicken legs prior to cooking). Data points represent the means and standard deviations of five independent readings (N = 5).

### DETAILED DESCRIPTION OF THE PREFERRED EMBODIMENT

Referring to the figures, **Figure 1****.** provides a schematic representation of the one-step adherent sandwich assay concept. In this concept, a DNA or possibly an RNA aptamer has been conjugated to a magnetic bead and used to capture a target analyte (bacterial cell in this example). Capture is achieved by specific aptamer binding to an epitope on the bacterial surface. Likewise, another epitope is bound by an aptamer-quantum dot conjugate or other aptamer-fluorophore reporter reagent simultaneously for fluorescent detection. Because the sandwich assay contains DNA or RNA, it is subject to adhering to some forms of charged glass or charged or uncharged plastics such as polystyrene and its derivatives by electrostatic and/or other weak forces such as dipole-dipole or Van der Waals interactions and possibly covalent electrophilic addition to alkenes or the styrene rings (Bensimon et al., 1994). Adherence is promoted by the addition of an external attractive magnetic force such as a strong Cobalt, Neodynium, or other rare earth magnet. After the external magnet is disengaged, the assay materials still adhere to the inner face of the cuvette due to interaction of DNA with the polystyrene or other plastic or glass materials. This adherence partitions the assay along with captured and labeled bacteria or other analytes from the bulk solution. If the solution is illuminated from the opposite side by an excitation source and the cuvette face with adherent assay materials is placed proximal to a photodetector, rapid, sensitive, one-step detection is enabled. Once adherence of all the aptamer-MB-bacteria-aptamer-QD complexes occurs on the surface, the adherent material emits a much brighter fluorescent signal than the bulk solution which contains free aptamer-QD or aptamer-fluorophore conjugates.

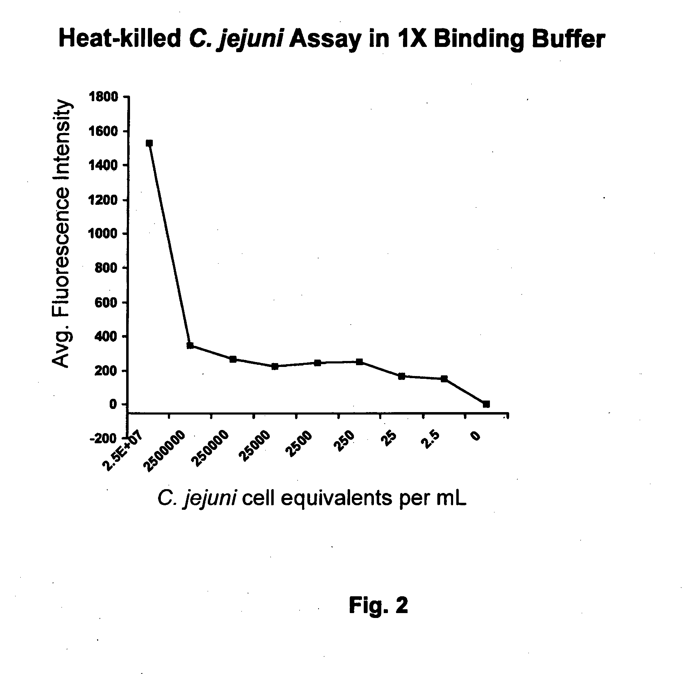

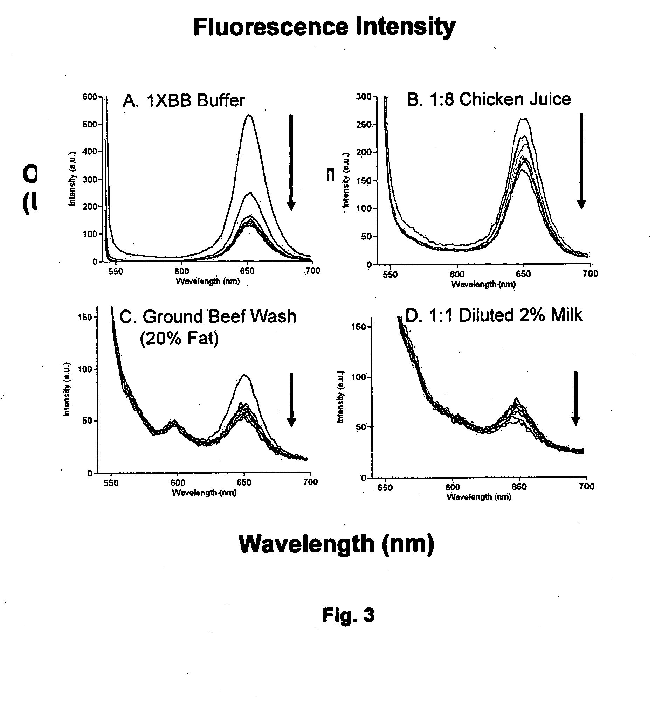

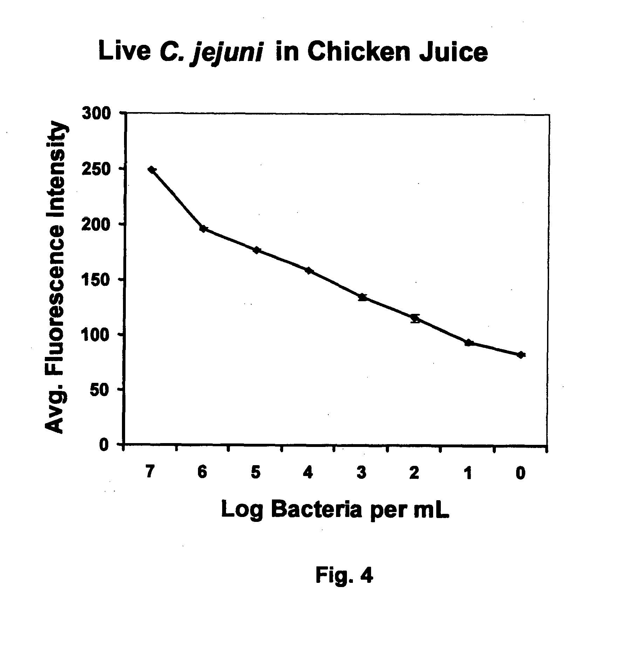

**Figure 2****.** shows line graphs plotting relative fluorescence intensity against the concentration *of Campylobacterjejuni (C. jejuni)* bacteria detected in neat buffer (1X binding buffer; 1XBB) down to a level of approximately 2 bacterial cells per milliliter using the one-step adherent DNA aptamer-MB-aptamer red QD (Q-dot 655 nm) sandwich assay without a wash step. Five independent readings were taken per data point with the green (Rhodamine) channel of a Turner Biosystems, Inc. handheld fluorometer. Error bars which are not visible due to their small numerical values represent the standard deviations of the 5 readings. The preferred embodiment for the adherent one-step washless aptamer-MB/aptamer-QD or aptamer-fluorophore assays is in a plastic polystyrene cuvette using lyophilized (freeze-dried) sandwich assay materials with long shelf-life that are rehydrated as needed. Their fluorescence can be assessed after a 15-20 minute capture and 5 minute magnetic collection period via a table top spectrofluorometer, or portable fluorometers such as the Turner Biosystem's Picofluor^{™} or Invitrogen's Q-Bit^{™} or other such fluorescence reader devices. The primarily linear photoresponse to logarithmic changes in bacterial concentration seen in Figures 2 and 4 is probably attributable to a photodiode detector in the fluorometer, such as is found in the Picofluor^{™}, versus the more sensitive and exponentially responsive photomultiplier tube (PMT) used for data collection by a spectrofluorometer in Figure 3.

**Figure 3****.** shows a series of fluorescence emission spectra from ten-fold serial dilutions of 25 million heat-killed *C. jejuni* bacteria per ml (highest peak) to 2.5 bacteria per ml and then zero bacteria per ml (lowest peak) detected by use of a Cary-Varian spectrofluorometer and the one-step plastic-adherent aptamer-MB/aptamer-red QD (Q-dot 655 nm) sandwich assay without a wash step directly in various food matrices as indicated. The arrows indicate the direction of increasing 2-fold dilutions or decreasing bacterial concentration. The assays are generally described herein as using a fluorescence intensity reporter method, which is a simple measure of fluorescence brightness, for detecting and quantifying the analyte-aptamer conjugate. Alternatively, the fluorescence intensity reporter method may be substituted by time-resolved fluorescence, chemiluminescence, electrical detection, electrochemical detection, electrochemiluminescence, phosphorescence, or radioisotopic detection instead of simple fluorescence intensity-based detection.

**Figure 4****.** illustrates a typical one-step assay capable of detecting 10 live *C. jejuni* bacteria in chicken juice (collected blood and fat globules from a fresh grocery store chicken product). Five independent readings were taken per data point with the green (Rhodamine) channel of a Turner Biosystems, Inc. handheld Picofluor^{™} fluorometer. Error bars which are barely visible due to their small numerical values represent the standard deviations of the 5 readings.

No wash steps are required and detection can be achieved directly in various food, environmental, or body fluid matrices as illustrated in Figures 3 and 4.

### Example 1

### One-Step, Washless Ultra sensitive Detection of Campylobacter jejuni in Various Food Matrices.

The invention has been used to detect as few as 2 live or dead *C. jejuni* bacterial cells (a common foodborne pathogen) in neat buffer and various food matrices as shown in Figures 2 - 4. In these assays, two different *C. jejuni* Sequences (designated C2 and C3 or SEQ ID NOs 2 and 3) were 5'-amine modified during solid-phase DNA synthesis and attached to either 1,000 tosyl-M280 (2.8 micron diameter) Dynal (Invitrogen, Inc.) MBs or 0.24 picoliters of Q-dot 655 ITK reagent (Invitrogen, Inc.) per test. The C2 aptamer (SEQ ID NO. 2) was used for capture on the surface of tosyl-MBs and the C3 aptamer (SEQ ID NO. 3) was used as the reporter reagent after attachment to the Q-dot 655 ITK reagent via BS³ (bis-suberate bifunctional linker from Pierce Chemical Co.). The reagents were purified, mixed together and lyophilized in plastic cuvettes. The powdered assays were later backflushed with nitrogen and capped. Upon rehydration, the adherent one-step sandwich assays were used to detect live or dead (heat-killed) *C. jejuni* cells with the very sensitive results depicted in Figures 2 - 4. Other aptamers chosen for capture and reporter functions from SEQ ID NOs. 1-6 can be substituted in *Campylobacter* assays which are functional, but result in somewhat less sensitive detection.

### Example 2

### One-Step, Washless Ultrasensitive Detection of Escherichia coli O157:H7 and other toxigenic (Shiga or Verotoxin-producing) E. coli in Various Food Matrices.

The present invention has potential to be used for detection of enterohemorraghic *E. coli* O157:H7 in and on various foods via binding of aptamers to the outer saccharides of 0157 lipopolysaccharide (LPS) and the H7 flagellar antigen. Aptamer sequences from SEQ ID NOs. 7-20 could be chosen for capture (aptamer-MB conjugate) or reporter (aptamer-fluorophore conjugate) functions and used to detect *E. coli* O157:H7 in or on foods. Alternatively, outer membrane proteins (OMPs) common to many species of *E. coli* can be used for aptamer-MB-based capture (or identification) of the *E. coli* bacterial cells followed by specific identification of the *E. coli* strain or serotype using LPS-specific aptamer-QD reporter reagents to complete the sandwich assay. Aptamer SEQ ID NOs. 279-322 can be used for *E. coli* OMP recognition and capture. In yet another embodiment non-O157:H7 toxigenic *E. coli* bacteria can be sensitively identified by their secretion of Shiga or Verotoxins (types 1 and 2 or Stx-1 and Stx-2). Many other strains of *E. coli* including 0126 can produce deadly disease in humans and the common thread among these lethal pathogens is the secretion of Stx. Therefore, a very useful embodiment of the invention would be detection of Stx-1 and/or Stx-2 using any of the DNA aptamer sequences identified by SEQ ID NOs. 323-352.

### Example 3

### One-Step, Washless Ultrasensitive Detection of Listeria monocytogenes in Various Food Matrices.

The present invention has potential to be used for detection of lethal *L. monocytogenes* in and on various foods via binding to the listerolysin (LO) surface protein. Aptamer sequences from SEQ ID NOs. 21-52 could be chosen for capture (aptamer-MB conjugate) or reporter (aptamer-fluorophore conjugate) functions and used to detect LO and *L. moncytogenes* in or on foods.

### Example 4

### One-Step, Washless Ultrasensitive Detection of Salmonella typhimurium in Various Food Matrices.

The present invention has potential to be used for detection of *S. typhimurium* and other *Salmonella* species *(S. typhi* etc.) in and on various foods. *S. typhimurium* has been renamed *Salmonella enterica* serovar Typhimurium, but many microbiologists and lay people still refer to the microbe as *S. typhimurium.* Aptamer sequences from SEQ ID NOs. 53-68 could be chosen for capture (aptamer-MB conjugate) or reporter (aptamer-fluorophore conjugate) functions for detection of *Salmonella typhimurium* LPS bacteria in or on foods. In addition, aptamer SEQ ID NOs. 353-392 could be used for capture or identification *of S. typhimurium* OMPs. These *S. typhimurium* DNA aptamer sequences are unique and bear no resemblance to those recently reported by Joshi et al. (2008).

### Example 5

### Detection of Fecal Contamination of Water Supplies

The present invention has the potential to detect all species of *Escherichia coli* bacteria in recreational, treated waste water, and drinking water supplies using aptamer DNA SEQ ID NOs. 69-122 directed against common core components of LPS for capture and reporter functions. The present invention has the potential to detect all species of *Enterococcus* bacteria (another common fecal indicator organism) in recreational, treated waste water, and drinking water supplies using aptamer DNA SEQ ID NOs. 123-130 directed against common teichoic acid moieties for capture and reporter functions.

### Example 6

### Detection of Leishmania Parasites in Skin Lesions or Visceral Fluids

The present invention has the potential to detect *Leishmania donovani* or *L. tropica* parasites in skin lesions of body fluids and other samples using aptamer DNA Sequences chosen from SEQ ID NOs. 131-134 directed against surface proteins of common to both *Leishmania* species for capture and reporter functions.

### Example 7

### Detection of Encapsultaed Vegetative Bacillus anthracis Bacteria in Blood and Body Fluids

The invention has the potential to detect encapsulated *B. anthracis* (anthrax) vegetative bacteria in blood and body fluids and other samples using aptamer DNA Sequences chosen from SEQ ID NOs. 135-138 directed against surface poly-D-glutamic acid (PDGA) capsular materials for capture and reporter functions.

### Example 8

### Detection of Small Molecules (< 1,000 Daltons) in Environmental and Clinical Samples

The invention has the potential to detect small molecules of < 1,000 Daltons, if the target has two distinct and accessible epitopes for attachment of capture and reported aptamers to enable a sandwich assay format. Among such small molecule targets would be organophosphorus pesticides (such as diazinon and malathion) in environmental water, soil, or mud samples as well as blood and body fluids and other samples using aptamer DNA Sequences chosen from SEQ ID NOs. 139-154 directed against different ends of the pesticide molecule for capture and reporter functions. In addition, vitamins such as 25-hydroxyvitamin D₃ (calcidiol; SEQ ID NOs. 243-274), the neurotransmitter acetylcholine (ACh; SEQ ID Nos. 393-416) might be viable targets for this novel adherent assay format

### Example 9

### Detection of Foot-and-Mouth Disease (FMD) and Related Viruses

The invention has the potential to detect FMD and related viruses in blood and body fluids and other samples using aptamer DNA Sequences chosen from SEQ ID NOs. 155-164 directed against a conserved 16-amino acid peptide from several O serotypes of FMD for capture and reporter functions.

### Example 10

### Detection of Bone Resorption or Syntheses Markers in Blood and Body Fluids

The invention has the potential to detect markers of bone loss such as cathepsin K, C-terminal telopeptides (CTx) and N-terminal telopeptides (NTx) of collagen, hydroxylysine (HL), osteocalcin fragments (OCF), etc. due to the effects of low gravity during lengthy spaceflights or osteoporosis and aging in blood, urine and other body fluids and other samples using aptamer DNA Sequences chosen from SEQ ID NOs. 165-242 directed against unique epitopes on each type of bone marker. The invention also has the potential to detect and discriminate various isomers of vitamin D associated with bone formation chosen from SEQ ID NOs. 243-274 for capture and reporter functions.

### Example 11

### Detection of Botulinum Toxin A and Related Biotoxins

The invention has the potential to detect *Clostridum botulinum* toxins which affect humans and animals (serotypes A-F) and related bacterial, harmful algal bloom (HAB, dinoflagellate), marine (shellfish-related), or plant toxins such as tetanus toxin, cholera and diphtheria toxins, shiga and verotoxins, staphylococcal enterotoxins, cyanotoxins, azaspiracids, brevetoxins, ciguatoxins, gonyautotoxins, domoic acid isomers, maitotoxins, palytoxins, yessotoxins, saxitoxins, ricin, gelonin, abrin, spider and snake venoms, etc. in blood and body fluids and other samples using aptamer DNA sequences in the adherent sandwich format. Aptamer sequences chosen from SEQ ID NOs. 275-278 in particular can be used to for detection of botulinum type A light chains or the holotoxin.

### Example 12

### Detection of Quorum Sensing Molecules in Bacterial Infections

Many species of bacteria are now known to communicate chemically via secreted small molecules. Many Gram negative bacterial pathogens commonly use a family of small molecules called acylhomoserine lactones (AHLs) to communicate between bacterial cells to sense when a critical concentration of cells or "quorum" has been reached to enable effective infection of a host organism. AHLs control induction of pathogenesis and virulence factors such as expression of adherence proteins and toxins. Therefore, early sensing of AHLs could indicate an imminent Gram negative bacterial infection and prompt a physician to administer the appropriate antibiotics to prevent an infection or more severe sepsis. AHLs do commonly possess two different ends or potential epitopes and are therefore potential candidates for the one-step plastic-adherent DNA aptamer-MB-aptamer-QD or other aptamer-reporter sandwich assays described herein. Sequence ID Nos. 417-426 illustrate potential aptamer DNA sequences developed against and reactive with the family of Gram negative bacterial AHLs for diagnostics.

**Table 1-DNA Aptamer Sequence ID Nos.**

| ***Campylobacter jejuni* Surface Protein Aptamers** |
|---|
| SEQ ID NO. 1 (C1) |
| CATCCGTCACACCTGCTCTGGGGAGGGTGGCGCCCGTCTCGGTGGTGTTGGCTCC CGTATCA |
| SEQ ID NO. 2 (C2) |
| CATCCGTCACACCTGCTCTGGGATAGGGTCTCGTGCTAGATGTGGTGTTGGCTCC CGTATCA |
| SEQ ID NO. 3 (C3) |
| CATCCGTCACACCTGCTCTGGACCGGCGCTTATTCCTGCTTGTGGTGTTGGCTCCC GTATCA |
| SEQ ID NO. 4 (C4) |
| CATCCGTCACACCTGCYCTGGAGCTGATATTGGATGGTCCGGTGGTGTTGGCTCC CGTATCA |
| SEQ ID NO. 5 (C5) |
| CATCCGTCACACCTGCYCYGCCCAGAGCAGGTGTGACGGATGTGGTGTTGGCTCC CGTATCA |
| SEQ ID NO. 6 (C6) |
| CATCCGTCACACCTGCYCYGCCGGACCATCCAATATCAGCTGTGGTGTTGGCTCC CGTATCA |

| ***E. coli* O157 Lipopolysaccharide (LPS) Aptamers** |
|---|
| SEQ ID NO. 7 (E-5F) |
| ATCCGTCACACCTGCTCTGGTGGAATGGACTAAGCTAGCTAGCGTTTTAAAAGGT GGTGTTGGCTCCCGTAT |
| SEQ ID NO. 8 (E-11F) |
| ATCCGTCACACCTGCTCTGTAAGGGGGGGAATCGCTTTCGTCTTAAGATGACAT GGTGTTGGCTCCCGTAT |
| SEQ ID NO.9 (E-12F) |
| ATCCGTCACACCTGCTCTGCCGGACCATCCAATATCAGCTGTGGTGTTGGCTCCC GTAT |
| SEQ ID NO. 10 (E-16F) |
| ATCCGTCACACCTGCTCTATCCGTCACGCCTGCTCTATCCGTCACACCTGCTCTGG TGTTGGCTCCCGTAT |
| SEQ ID NO. 11 (E-17F) |
| ATCCGTCACACCTGCTCTATCAAATGTGCAGATATCAAGACGATTTGTACAAGAT GGTGTTGGCTCCCGTAT |
| SEQ ID NO. 12 (E-18F) |
| ATCCGTCACACCTGCTCTGTAGATGGCAAGGCATAAGCGTCCGGAACGATAGAA TGGTGTTGGCTCCCGTAT |
| SEQ ID NO. 13 (E-19F) |
| ATCCGTCACACCTGCTCTGTAGATGGCAAGGCATAAGCGTCCGGAACGATAGAA TGGTGTTGGCTCCCGTAT |
| SEQ ID NO. 14 (E-5R) |
| ATACGGGAGCCAACACCACCTTTTAAAACGCTAGCTAGCTTAGTCCATTCCACCA GAGCAGGTGTGACGGAT |
| SEQ ID NO.15 (E-11R) |
| ATACGGGAGCCAACACCATGTCATCTTAAGACGAAAGCGATTCCCCCCCCTTACA GAGCAGGTGTGACGGAT |
| SEQ ID NO. 16 (E-12R) |
| ATACGGGAGCCAACACCACAGCTGATATTGGATGGTCCGGCAGAGCAGGTGTGA CGGAT |
| SEQ ID NO. 17 (E-16R) |
| ATACGGGAGCCAACACCAGAGCAGGTGTGACGGATAGAGCAGGCGTGACGGAT AGAGCAGGTGTGACGGAT |
| SEQ ID NO. 18 (E-17R) |
| ATACGGGAGCCAACACCATCTTGTACAAATCGTCTTGATATCTGCACATTTGATA GAGCAGGTGTGACGGAT |
| SEQ ID NO. 19 (E-18R) |
| ATACGGGAGCCAACACCATTCTATCGTTCCGGACGCTTATGCCTTGCCATCTACA GAGCAGGTGTGACGGAT |
| SEQ ID NO. 20 (E-19R) |
| ATACGGGAGCCAACACCATTCTATCGTTCCGGACGCTTATGCCTTGCCATCTACA GAGCAGGTGTGACGGAT |

| **Listeriolysin (A surface protein on *Listeria monocytogenes*) Aptamers** |
|---|
| SEQ ID NO. 21 (LO-10F) |
| ATCCGTCACACCTGCTCTGCCGGACCATCCAATATCAGCTGTGGTGTTGGCTCCC GTAT |
| SEQ ID NO. 22 (LO-11F) |
| ATCCGTCACACCTGCTCTGGTGGAATGGACTAAGCTAGCTAGCGTTTTAAAAGGT GGTGTTGGCTCCCGTAT |
| SEQ ID NO. 23 (LO-13F) |
| ATCCGTCACACCTGCTCTTAAAGTAGAGGCTGTTCTCCAGACGTCGCAGGAGGAT GGTGTTGGCTCCCGTAT |
| SEQ ID NO. 24 (LO-15F) |
| ATCCGTCACACCTGCTCTGTAGATGGCAAGGCATAAGCGTCCGGAACGATAGAA TGGTGTTGGCTCCCGTAT |
| SEQ ID NO. 25 (LO-16F) |
| ATCCGTCACACCTGCTCTGTAGATGGCAAGGCATAAGCGTCCGGAACGATAGAA TGGTGTTGGCTCCCGTAT |
| SEQ ID NO. 26 (LO-17F) |
| ATACGGGAGCCAACACCA CAGCTGATATTGGATGGTCCGGCAGAGCAGGTGTGACGGAT |
| SEQ ID NO. 27 (LO-19F) |
| ATCCGTCACACCTGCTCTTGGGCAGGAGCGAGAGACTCTAATGGTAAGCAAGAA TGGTGTTGGCTCCCGTAT |
| SEQ ID NO. 28 (LO-20F) |
| ATCCGTCACACCTGCTCTCCAACAAGGCGACCGACCGCATGCAGATAGCCAGGTT GGTGTTGGCTCCCGTAT |
| SEQ ID NO. 29 (LO-10R) |
| ATACGGGAGCCAACACCACAGCTGATATTGGATGGTCCGGCAGAGCAGGTGTGA CGGAT |
| SEQ ID NO. 30 (LO-11R) |
| ATACGGGAGCCAACACCACCTTTTAAAACGCTAGCTAGCTTAGTCCATTCCACCA GAGCAGGTGTGACGGAT |
| SEQ ID NO. 31 (LO-13R) |
| ATACGGGAGCCAACACCATCCTCCTGCGACGTCTGGAGAACAGCCTCTACTTTAA GAGCAGGTGTGACGGAT |
| SEQ ID NO. 32 (LO-15R) |
| ATACGGGAGCCAACACCATTCTATCGTTCCGGACGCTTATGCCTTGCCATCTACA GAGCAGGTGTGACGGAT |
| SEQ ID NO. 33 (LO-16R) |
| ATACGGGAGCCAACACCATTCTATCGTTCCGGACGCTTATGCCTTGCCATCTACA GAGCAGGTGTGACGGAT |
| SEQ ID NO. 34 (LO-17R) |
| ATCCGTCACACCTGCTCTGCCGGACCATCCAATATCAGCTGTGGTGTTGGCTCCC GTAT |
| SEQ ID NO. 35 (LO-19R) |
| ATACGGGAGCCAACACCATTCTTGCTTACCATTAGAGTCTCTCGCTCCTGCCCAA GAGCAGGTGTGACGGAT |
| SEQ ID NO. 36 (LO-20R) |
| ATACGGGAGCCAACACCAACCTGGCTATCTGCATGCGGTCGGTCGCCTTGTTGGA GAGCAGGTGTGACGGAT |

| **Listeriolysin (Alternate form of *Listeria* surface protein designated "Pest-Free") Aptamers** |
|---|
| SEQ ID NO. 37 (LP-3F) |
| ATCCGTCACACCTGCTCTGTAGATGGCAAGGCATAAGCGTCCGGAACGATAGAA TGGTGTTGGCTCCCGTAT |
| SEQ ID NO. 38 (LP-11F) |
| ATCCGTCACACCTGCTCTAACCAAAAGGGTAGGAGACCAAGCTAGCGATTTGGA TGGTGTTGGCTCCCGTAT |
| SEQ ID NO. 39 (LP-13F) |
| ATCCGTCACACCTGCTCTGCCGGACCATCCAATATCAGCTGTGGTGTTGGCTCCC GTAT |
| SEQ ID NO. 40 (LF-14F) |
| ATCCGTCACACCTGCTCTGAAGCCTAACGGAGAAGATGGCCCTACTGCCGTAGGT GGTGTTGGCTCCCGTAT |
| SEQ ID NO. 41 (LP-15F) |
| ATCCGTCACACCTGCTCTACTAAACAAGGGCAAACTGTAAACACAGTAGGGGCG TGGTGTTGGCTCCCGTAT |
| SEQ ID NO. 42 (LP-17F) |
| ATCCGTCACACCTGCTCTGGTGTTGGCTCCCGTATAGCTTGGCTCCCGTATGGTGT TGGCTCCCGTAT |
| SEQ ID NO. 43 (LP-18F) |
| ATCCGTCACACCTGCTCTGTCGCGATGATGAGCAGCAGCGCAGGAGGGAGGGGG TGGTGTTGGCTCCCGTAT |
| SEQ ID NO. 44 (LP-20F) |
| ATCCGTCACACCTGCTCTGATCAGGGAAGACGCCAACACTGGTGTTGGCTCCCGT AT |
| SEQ ID NO. 45 (LP-3R) |
| ATACGGGAGCCAACACCATTCTATCGTTCCGGACGCTTATGCCTTGCCATCTACA GAGCAGGTGTGACGGAT |
| SEQ ID NO. 46 (LP-11R) |
| ATACGGGAGCCAACACCATCCAAATCGCTAGCTTGGTCTCCTACCCTTTTGGTTA GAGCAGGTGTGACGGAT |
| SEQ ID NO. 47 (LP-13R) |
| ATACGGGAGCCAACACCACAGCTGATATTGGATGGTCCGGCAGAGCAGGTGTGA CGGAT |
| SEQ ID NO. 48 (LP-14R) |
| ATACGGGAGCCAACACCACCTACGGCAGTAGGGCCATCTTCTCCGTTAGGCTTCA GAGCAGGTGTGACGGAT |
| SEQ ID NO. 49 (LP-15R) |
| ATACGGGAGCCAACACCACGCCCCTACTGTGTTTACAGTTTGCCCTTGTTTAGTA GAGCAGGTGTGACGGAT |
| SEQ ID NO. 50 (LP-17R) |
| ATACGGGAGCCAACACCATACGGGAGCCAAGCTATACGGGAGCCAACACCAGA GCAGGTGTGACGGAT |
| SEQ ID NO. 51 (LP-18R) |
| ATACGGGAGCCAACACCACCCCCTCCCTCCTGCGCTGCTGCTCATCATCGCGACA GAGCAGGTGTGACGGAT |
| SEQ ID NO. 52 (LP-20R) |
| ATACGGGAGCCAACACCAGTGTTGGCGTCTTCCCTGATCAGAGCAGGTGTGACG GAT |

| ***Salmonella typhimurium* lipopolysaccharide (LPS) Aptamers** |
|---|
| SEQ ID NO. 53 (St-7F) |
| ATCCGTCACACCTGCTCTGTCCAAAGGCTACGCGTTAACGTGGTGTTGGCTCCCG TAT |
| SEQ ID NO. 54 (St-10F) |
| ATCCGTCACACCTGCTCTGGAGCAATATGGTGGAGAAACGTGGTGTTGGCTCCCG TAT |
| SEQ ID NO. 55 (St-11F) |
| ATCCGTCACACCTGCTCTGCCGGACCATCCAATATCAGCTGTGGTGTTGGCTCCC GTAT |
| SEQ ID NO. 56 (St-15F) |
| ATCCGTCACACCTGCTCTGAACAGGATAGGGATTAGCGAGTCAACTAAGCAGCA TGGTGTTGGCTCCCGTAT |
| SEQ ID NO. 57 (St-16F) |
| ATCCGTCACACCTGCTCTGGCGGACAGGAAATAAGAATGAACGCAAAATTTATC TGGTGTTGGCTCCCGTAT |
| SEQ ID NO. 58 (St-18F) |
| ATCCGTCACACCTGCTCTACGCAACGCGACAGGAACATTCATTATAGAATGTGTT GGTGTTGGCTCCCGTAT |
| SEQ ID NO. 59 (St-19F) |
| ATCCGTCACACCTGCTCTCGGCTGCAATGCGGAGAGTAGGGGGGAACCAAACC TGGTGTTGGCTCCCGTAT |
| SEQ ID NO. 60 (St-20F) |
| ATCCGTCACACCTGCTCTATGACTGGAACACGGGTATCGATGATTAGATGTCCTT GGTGTTGGCTCCCGTAT |
| SEQ ID NO. 61 (St-7R) |
| ATACGGGAGCCAACACCACGTTAACGCGTAGCCTTTGGACAGAGCAGGTGTGAC GGAT |
| SEQ ID NO. 62 (St-10R) |
| ATACGGGAGCCAACACCACGTTTCTCCACCATATTGCTCCAGAGCAGGTGTGACG GAT |
| SEQ ID NO. 63 (St-11R) |
| ATACGGGAGCCAACACCACAGCTGATATTGGATGGTCCGGCAGAGCAGGTGTGA CGGAT |
| SEQ ID NO. 64 (St-15R) |
| ATACGGGAGCCAACACCATGCTGCTTAGTTGACTCGCTAATCCCTATCCTGTTCA GAGCAGGTGTGACGGAT |
| SEQ ID NO. 65 (St-16R) |
| ATACGGGAGCCAACACCAGATAAATTTTGCGTTCATTCTTATTTCCTGTCCGCCA GAGCAGGTGTGACGGAT |
| SEQ ID NO. 66 (St-18R) |
| ATACGGGAGCCAACACCAACACATTCTATAATGAATGTTCCTGTCGCGTTGCGTA GAGCAGGTGTGACGGAT |
| SEQ ID NO. 67 (St-19R) |
| ATACGGGAGCCAACACCAGGTTTGGTTCCCCCCTACTCTCCCGCATTGCAGCCGA GAGCAGGTGTGACGGAT |
| SEQ ID NO. 68 (St-20R) |
| ATACGGGAGCCAACACCAAGGACATCTAATCATCGATACCCGTGTTCCAGTCATA GAGCAGGTGTGACGGAT |

| **Fecal Contamination Indicator (Core LPS Antigens) Aptamers** |
|---|
| SEQ ID NO. 69 (Glucosamine(G)1F) |
| ATCCGTCACACCTGCTCTAATTAGGATACGGGGCAACAGAACGAG AGGGGGGAATGGTGTTGGCTCCCGTAT |
| SEQ ID NO. 70 (G2F) |
| ATCCGTCACACCTGCTCTCGGACCAGGTCAGACAAGCACATCGGAT ATCCGGCTGGTGTTGGCTCCCGTAT |
| SEQ ID NO. 71 (G5F) |
| ATCCGTCACACCTGCTCTTGAGTCAAAGAGTTTAGGGAGGAGCTAA CATAACAGTGGTGTTGGCTCCCGTAT |
| SEQ ID NO. 72 (G7F) |
| ATCCGTCACACCTGCTCTAACAACAATGCATCAGCGGGCTGGGAAC GCATGCGGTGGTGTTGGCTCCCGTAT |
| SEQ ID NO. 73 (G8F) |
| ATCCGTCACACCTGCTCTGAACAGGTTATAAGCAGGAGTGATAGTT TCAGGATCTGGTGTTGGCTCCCGTAT |
| SEQ ID NO. 74 (G9F) |
| ATCCGTCACACCTGCTCTCGGCGGCTCGCAAACCGAGTGGTCAGCA CCCGGGTTGGTGTTGGCTCCCGTAT |
| SEQ ID NO. 75 (G10F) |
| ATCCGTCACACCTGCTCTGCGCAAGACGTAATCCACAAGACCGTGA AAACATAGTGGTGTTGGCTCCCGTAT |
| SEQ ID NO. 76 (G1R) |
| ATACGGGAGCCAACACCATTCCCCCCTCTCGTTCTGTTGCCCCGTAT CCTAATTAGAGCAGGTGTGACGGAT |
| SEQ ID NO. 77 (G2R) |
| ATACGGGAGCCAACACCAGCCGGATATCCGATGTGCTTGTCTGACC TGGTCCGAGAGCAGGTGTGACGGAT |
| SEQ ID NO. 78 (G5R) |
| ATACGGGAGCCAACACCACTGTTATGTTAGCTCCTCCCTAAACTCT TTGACTCAAGAGCAGGTGTGACGGAT |
| SEQ ID NO. 79 (G7R) |
| ATACGGGAGCCAACACCACCGCATGCGTTCCCAGCCCGCTGATGCA TTGTTGTTAGAGCAGGTGTGACGGAT |
| SEQ ID NO. 80 (G8R) |
| ATACGGGAGCCAACACCAGATCCTGAAACTATCACTCCTGCTTATA ACCTGTTCAGAGCAGGTGTGACGGAT |
| SEQ ID NO. 81 (G9R) |
| ATACGGGAGCCAACACCAACCCGGGTGCTGACCACTCGGTTTGCGA GCCGCCGAGAGCAGGTGTGACGGAT |
| SEQ ID NO. 82 (G10R) |
| ATACGGGAGCCAACACCACTATGTTTCACGGTCTTGTGGATTACG TCTTGCGCAGAGCAGGTGTGACGGAT |
| SEQ ID NO. 83 (KDO (K) Antigen 2F) |
| ATCCGTCACACCTGCTCTAGGCGTAGTGACTAAGTCGCGAAAAT CACAGCATTGGTGTTGGCTCCCGTAT |
| SEQ ID NO. 84 (K5F) |
| ATCCGTCACACCTGCTCTCAGCGGCAGCTATACAGTGAGAACGGAC TAGTGCGTTGGTGTTGGCTCCCGTAT |
| SEQ ID NO. 85 (K7F) |
| ATCCGTCACACCTGCTCTGGCAAATAATACTAGCGATGATGGATCT GGATAGACTGGTGTTGGCTCCCGTAT |
| SEQ ID NO. 86 (K8F) |
| ATCCGTCACACCTGCTCTGGGGGTGCGACTTAGGGTAAGTGGGAAA GACGATGCTGGTGTTGGCTCCCGTAT |
| SEQ ID NO. 87 (K9F) |
| ATCCGTCACACCTGCTCTCAAGAGGAGATGAACCAATCTTAGTCCG ACAGGCGGTGGTGTTGGCTCCCGTAT |
| SEQ ID NO. 88 (K10F) |
| ATCCGTCACACCTGCTCTGGCCCGGAATTGTCATGACGTCACCTAC ACCTCCTGTGGTGTTGGCTCCCGTAT |
| SEQ ID NO. 89 (K2R) |
| ATACGGGAGCCAACACCAATGCTGTGATTTCGCGACTTAGTCA CTACGCCTAGAGCAGGTGTGACGGAT |
| SEQ ID NO. 90 (K5R) |
| ATACGGGAGCCAACACCAACGCACTAGTCCGTTCTCACTGTATAGC TGCCGCTGAGAGCAGGTGTGACGGAT |
| SEQ ID NO. 91 (K7R) |
| ATACGGGAGCCAACACCAGTCTATCCAGATCCATCATCGCTAGTAT TATTTGCCAGAGCAGGTGTGACGGAT |
| SEQ ID NO. 92 (K8R) |
| ATACGGGAGCCAACACCAGCATCGTCTTTCCCACTTACCCTAAGTC GCACCCCCAGAGCAGGTGTGACGGAT |
| SEQ ID NO. 93 (K9R) |
| ATACGGGAGCCAACACCACCGCCTGTCGGACTAAGATTGGTTCATC TCCTCTTGAGAGCAGGTGTGACGGAT |
| SEQ ID NO. 94 (K10R) |
| ATACGGGAGCCAACACCACAGGAGGTGTAGGTGACGTCATGACAA TTCCGGGCCAGAGCAGGTGTGACGGAT |
| SEQ ID NO. 95 (Whole LPS from *E. coli* O111:B4 (L)1F) |
| ATCCGTCACCCCTGCTCTCGTCGCTATGAAGTAACAAAGATAGGAG CAATCGGGTGGTGTTGGCTCCCGTAT |
| SEQ ID NO. 96 (L3F) |
| ATCCGTCACACCTGCTCTAACGAAGACTGAAACCAAAGCAGTGAC AGTGCTGAATGGTGTTGGCTCCCGTAT |
| SEQ ID NO. 97 (L4F) |
| ATCCGTCACACCTGCTCTCGGTGACAATAGCTCGATCAGCCCAAAG TCGTCAGATGGTGTTGGCTCCCGTAT |
| SEQ ID NO. 98 (L6F) |
| ATCCGTCACACCTGCTCTAACGAAATAGACCACAAATCGATACTTT ATGTTATTGGTGTTGGCTCCCGTAT (71) |
| SEQ ID NO. 99 (L7F) |
| ATCCGTCACACCTGCTCTGTCGAATGCTCTGCCTGGAAGAGTTGTT AGCAGGGATGGTGTTGGCTCCCGTAT |
| SEQ ID NO. 100 (L8F) |
| ATCCGTCACACCTGCTCTTAAGCCGAGGGGTAAATCTAGGACAGGG GTCCATGATGGTGTTGGCTCCCGTAT |
| SEQ ID NO. 101 (L9F) |
| ATCCGTCACACCTGCTCTACTGGCCGGCTCAGCATGACTAAGAAGG AAGTTATGTGGTGTTGGCTCCCGTAT |
| SEQ ID NO. 102 (L10F) |
| ATCCGTCACACCTGCTCTGGTACGAATCACAGGGGATGCTGGAAGC TTGGCTCTTGGTGTTGGCTCCCGTAT |
| SEQ ID NO. 103 (L1R) |
| ATACGGGAGCCAACACCACCCGATTGCTCCTATCTTTGTTACTTCAT AGCGACGAGAGCAGGGGTGACGGAT |
| SEQ ID NO. 104 (L3R) |
| ATACGGGAGCCAACACCATTCAGCACTGTCACTGCTTTGGTTTCAG TCTTCGTTAGAGCAGGTGTGACGGAT |
| SEQ ID NO. 105 (L4R) |
| ATACGGGAGCCAACACCATCTGACGACTTTGGGCTGATCGAGCTAT TGTCACCGAGAGCAGGTGTGACGGAT |
| SEQ ID NO. 106 (L6R) |
| ATACGGGAGCCAACACCAATAACATAAAGTATCGATTTGTGGTCTA TTTCGTTAGAGCAGGTGTGACGGAT |
| SEQ ID NO. 107 (L7R) |
| ATACGGGAGCCAACACCATCCCTGCTAACAACTCTTCCAGGCAGAG CATTCGACAGAGCAGGTGTGACGGAT |
| SEQ ID NO. 108 (L8R) |
| ATACGGGAGCCAACACCATCATGGACCCCTGTCCTAGATTTACCCC TCGGCTTAAGAGCAGGTGTGACGGAT |
| SEQ ID NO. 109 (L9R) |
| ATACGGGAGCCAACACCACATAACTTCCTTCTTAGTCATGCTGAGC CGGCCAGTAGAGCAGGTGTGACGGAT |
| SEQ ID NO. 110 (L10R) |
| ATACGGGAGCCAACACCAAGAGCCAAGCTTCCAGCATCCCCTGTG ATTCGTACCAGAGCAGGTGTGACGGAT |
| SEQ ID NO. 111 (Rough (Ra or R) Core LPS Antigens R1F) |
| ATCCGTCACACCTGCTCTCCGCACGTAGGACCACTTTGGTACACGC TCCCGTAGTGGTGTTGGCTCCCGTAT |
| SEQ ID NO. 112 (R5F) |
| ATCCGTCACACCTGCTCTACGGATGAACGAAGATTTTAAAGTCAAG CTAATGCATGGTGTTGGCTCCCGTAT |
| SEQ ID NO. 113 (R6F) |
| ATCCGTCACACCTGCTCTGTAGTGAAGAGTCCGCAGTCCACGCTGT TCAACTCATGGTGTTGGCTCCCGTAT |
| SEQ ID NO. 114 (R7F) |
| ATCCGTCACACCTGCTCTACCGGCTGGCACGGTTATGTGTGACGGG CGAAGATATGGTGTTGGCTCCCGTAT |
| SEQ ID NO. 115 (R9F) |
| ATCCGTCACACCTGCTCTGCGTGTGGAGCGCCTAGGTGAGTGGTGT TGGCTCCCGTAT |
| SEQ ID NO. 116 (R10F) |
| ATCCGTCACACCTGCTCTGATGTCCCTTTGAAGAGTTCCATGACGCT GGCTCCTTGGTGTTGGCTCCCGTAT |
| SEQ ID NO. 117 (R1R) |
| ATACGGGAGCCAACACCACTACGGGAGCGTGTACCAAAGTGGTCC TACGTGCGGAGAGCAGGTGTGACGGAT |
| SEQ ID NO. 118 (R5R) |
| ATACGGGAGCCAACACCATGCATTAGCTTGACTTTAAAATCTTCGT TCATCCGTAGAGCAGGTGTGACGGAT |
| SEQ ID NO. 119 (R6R) |
| ATACGGGAGCCAACACCATGAGTTGAACAGCGTGGACTGCGGACT CTTCACTACAGAGCAGGTGTGACGGAT |
| SEQ ID NO. 120 (R7R) |
| ATACGGGAGCCAACACCATATCTTCGCCCGTCACACATAACCGTGC CAGCCGGTAGAGCAGGTGTGACGGAT |
| SEQ ID NO. 121 (R9R) |
| ATACGGGAGCCAACACCACTCACCTAGGCGCTCCACACGCAGAGC AGGTGTGACGGAT |
| SEQ ID NO. 122 (R10R) |
| ATACGGGAGCCAACACCAAGGAGCCAGCGTCATGGAACTCTTCAA AGGGACATCAGAGCAGGTGTGACGGAT |

| ***Enterococcus faecalis* Teichoic Acid (TA) Aptamers** |
|---|
| SEQ ID NO. 123 (TA5F) |
| CATTCACCACACCTCTGCTGGCTTGGCTAGCCTTGATGCTAAACGACCCATAGTG TGGTGTCGTCCCGTATC |
| SEQ ID NO. 124 (TA5R) |
| GATACGGGACGACACCACACTATGGGTCGTTTAGCATCAAGGCTAGCCAAGCCA GCAGAGGTGTGGTGAATG |
| SEQ ID NO. 125 (TA6F) |
| CATTCACCACACCTCTGCTGGAGGAGGAAGTGGTCTGGAGTTACTTGACATAGTG TGGTGTCGTCCCGTATC |
| SEQ ID NO. 126 (TA6R) |
| GATACGGACGACACCACACTATGTCAAGTAACTCCAGACCACTTCCTCCTCCAG CAGAGGTGTGGTGAATG |
| SEQ ID NO. 127 (TA7F) |
| CATTCACCACACCTCTGCTGGACGGAAACAATCCCCGGGTACGAGAATCAGGGT GTGGTGTCGTCCCGTATC |
| SEQ ID NO. 128 (TA7R) |
| GATACGGGACGACACCACACCCTGATTCTCGTACCCGGGGATTGTTTCCGTCCAG CAGAGGTGTGGTGAATG |
| SEQ ID NO. 129 (TA9F) |
| CATTCACCACACCTCTGCTGGAAACCTACCATTAATGAGACATGATGCGGTGGTG TGGTGTCGTCCCGTATC |
| SEQ ID NO. 130 (TA9R) |
| GATACGGGACGACACCACACCACCGCATCATGTCTCATTAATGGTAGGTTTCCAG CAGAGGTGTGGTGAATG |

| ***Leishmania donovani* and *Leishmania tropica* Surface Protein Aptamers** |
|---|
| SEQ ID NO. 131 (L-3F) |
| GATACGGGAGCCAACACCACCCGTATCGTTCCCAATGCACTCAGAGCAGGTGTG ACGGATG |
| SEQ ID NO. 132 (L-3R) |
| CATCCGTCACACCTGCTCTGAGTGCATTGGGAACGATACGGGTGGTGTTGGCTCC CGTATG |
| SEQ ID NO. 133 (L-5F) |
| GATACGGGAGCCAACACCACGTTCCCATACAAGTTACTGACAGAGCAGGTGTGA CGGATG |
| SEQ ID NO. 134 (L-5R) |
| CATCCGTCACACCTGCTCTGTCAGTAACTTGTATGGGAACGTGGTGTTGGCTCCC GTATC |

| ***Bacillus anthracis* Poly-D-Glutamic Acid Capsule Aptamers** |
|---|
| SEQ ID NO. 135 (PDGA 2F) |
| CATCCGTCACACCTGCTCTGGTTCGCCCGGTCAAGGAGAGTGGTGTTGGCTCCC GTATC |
| SEQ ID NO. 136 (PDGA 2R) |
| GATACGGGAGCCAACACCACTCTCCTTGACCGGGGCGAACCAGAGCAGGTGTGA CGGATG |
| SEQ ID NO. 137 (PDGA 5F) |
| CATCCGTCACACCTGCTCTGGATAAGATCAGCAACAAGTTAGTGGTGTTGGCTCC CGTATC |
| SEQ ID NO. 138 (PDGA 5R) |
| GATACGGGAGCCAACACCACTAACTTGTTGCTGATCTTATCAGAGCAGGTGTGAC GGATG |

| **Organophosphorus Pesticide Aptamers** |
|---|
| SEQ ID NO. 139 (Diazinon (D)12F) |
| ATACGGGAGCCAACACCATTAAATCAATTGTGCCGTGTTGGTCTTGTCTCATCGA GAGCAGGTGTGACGGAT |
| SEQ ID NO. 140 (D12R) |
| ATCCGTCACACCTGCTCTCGATGAGACAAGACCAACACGGCACAATTGATTTAAT GGTGTTGGCTCCCGTAT |
| SEQ ID NO. 141 (D17F) |
| ATACGGGAGCCAACACCATTTTTATTATCGGTATGATCCTACGAGTTCCTCCCAA GAGCAGGTGTGACGGAT |
| SEQ ID NO. 142 (D17R) |
| ATCCGTCACACCTGCTCTTGGGAGGAACTCGTAGGATCATACCGATAATAAAAAT GGTGTTGGCTCCCGTAT |
| SEQ ID NO. 143 (D18F) |
| ATACGGGAGCCAACACCACGTATATCTTATTATGCACAGCATCACG AAAGTGC- AGAGCAGGTGTGACGGAT |
| SEQ ID NO. 144 (D18R) |
| ATCCGTCACACCTGCTCTTTTTATTATCGGTATGATCCTACGAGTTCCTCCCATG GTGTTGGCTCCCGTAT |
| SEQ ID NO. 145 (D19F) |
| ATACGGGAGCCAACACCATTAACGTTAAGCGGCCTCACTTCTTTTAATCCTTTCA GAGCAGGTGTGACGGAT |
| SEQ ID NO. 146 (D19R) |
| ATCCGTCACACCTGCTCTGAAAGGATTAAAAGAAGTGAGGCCGCTTAACGTTAAT GGTGTTGGCTCCCGTAT |
| SEQ ID NO. 147 (D20F) |
| ATCCGTCACACCTGCTCTAATATAGAGGTATTGCTCTTGGACAAGGTACAGGGAT GGTGTTGGCTCCCGTAT |
| SEQ ID NO. 148 (D20R) |
| ATACGGGAGCCAACACCATCCCTGTACCTTGTCCAAGAGCAATACCTCTATATTA CCACAACCGAGGGCATA |
| SEQ ID NO. 149 (Malathion (M)17F) |
| ATACGGGAGCCAACACCACAGTCAAGAAGTTAAGAGAAAAACAATTGTGTATAA GAGCAGGTGTGACGGAT |
| SEQ ID NO. 150 (M17R) |
| ATCCGTCACACCTGCTCTTATACACAATTGTTTTCTCTTAACTTCTTGACTGCTG GTGTTGGCTCCCGTAT |
| SEQ ID NO. 151 (M21F) |
| ATCCGTCACACCTGCTCTGCGCCACAAGATTGCGGAAAGACACCCGGGGGGCTT GGTGTTGGCTCCCGTAT |
| SEQ ID NO. 152 (M21R) |
| ATACGGGAGCCAACACCAGCCCCCGGGTGTCTTTCCGCAATCTTGTGGCGCAGA GCAGGTGTGACGGAT |
| SEQ ID NO. 153 (M25F) |
| ATCCGTCACACCTGCTCTGGCCTTATGTAAAGCGTTGGGTGGTGTTGGCTCCCGT AT |
| SEQ ID NO. 154 (M25R) |
| ATACGGGAGCCAACACCACCCAACGCTTTACATAAGGCCAGAGCAGGTGTGACG GAT |

| **Foot-and-Mouth Disease (FMD) O-Serotype Viral Capsid Aptamers** |
|---|
| SEQ ID NO. 155 (FMD 1F) |
| ATACGGGAGCCAACACCATTCTATCGTTCCGGACGCTTATGCCTTGCCATCTACA GAGCAGGTGTGACGGAT |
| SEQ ID NO. 156 (FMD 1R) |
| ATCCGTCACTCCTGCTCTGTAGATGGCAAGGCATAAGCGTCCGGAACGATAGAAT GGTGTTGGCTCCCGTAT |
| SEQ ID NO. 157 (FMD 10F) |
| ATACGGGAGCCAACACCATGAATATCTCTTCTACCTCCTCTCCTCCC TTTACTTAGAGCAGGTGTGACGGAT |
| SEQ ID NO. 158 (FMD 10R) |
| ATCCGTCACTCCTGCTCTAAGTAAAGGGAGGAGAGGAGGTAGAAGAGATATTCA TGGTGTTGGCTCCCGTAT |
| SEQ ID NO. 159 (FMD 11F) |
| ATACGGGAGCCAACACCACGCCGCCCCAGTTCATGGCCTCTATGTCCGGCAACG AGAGCAGGTGTGACGGAT |
| SEQ ID NO. 160 (FMD 11 R) |
| ATCCGTCACTCCTGCTCTCGTTGCCGGACATAGAGGCCATGAACTGGGGCGGCGT GGTGTTGGCTCCCGTAT |
| SEQ ID NO. 161 (FMD 12F) |
| ATACGGGAGCCAACACCATCTAGATCTGAAGAATAAAACAAAGACAAAGATGCT AGAGCAGGTGTGACGGAT |
| SEQ ID NO. 162 (FMD 12R) |
| ATCCGTCACTCCTGCTCTAGCATCTTTGTCTTTGTTTATTCAGATCTAGATGGTG TTGGCTCCCGTAT |
| SEQ ID NO. 163 (FMD 13F) |
| ATACGGGAGCCAACACCACCTTTTAAAACGCTAGCTAGCTTAGTCCATTCCACCA GAGCAGGTGTGACGGAT |
| SEQ ID NO. 164 (FMD 13R) |
| ATCCGTCACTCCTGCTCTGGTGGAATGGACTAAGCTAGCTAGCGTTTTAAAAGGT GGTGTTGGCTCCCGTAT |

| **Aptamer Sequences Against Markers of Bone Resorption or Formation** |
|---|
| SEQ ID NO. 165 (Hydroxylysine 5F) |
| ATACGGGAGCCAACACCACGCTTAGATATTATCCTTGTCCAGAGCA GGTGTGACGGAT |
| SEQ ID NO. 166 (Hydroxylysine 5R) |
| ATCCGTCACACCTGCTCTGGACAAGGATAATATCTAAGCGTGGTGT TGGCTCCCGTAT |
| SEQ ID NO. 167 (Hydroxylysine 6F) |
| ACACGGGAGCCAACACCATCCATAGCTCATCTATACCCTCTTCCGA GTCCCACCAGAGCAGGTGTGACGGAT |
| SEQ ID NO. 168 (Hydroxylysine 6R) |
| ATCCGTCACACCTGCTCTGGTGGGACTCGGAAGAGGGTATAGATGA GCTATGGATGGTGTTGGCTCCCGTGT |
| SEQ ID NO. 169 (Hydroxylysine 7F) |
| ATACGGGAGCCAACACCACCCTACACCAGCGCCCTACACTTTTGTA GCACTTCGAGAGCAGGTGTGACGGAT |
| SEQ ID NO. 170 (Hydroxylysine 7R) |
| ATCCGTCACACCTGCTCTCGAAGTGCTACAAAAGTGTAGGGCGCTG GTGTAGGGTGGTGTTGGCTCCCGTAT |
| SEQ ID NO. 171 (Hydroxylysine 8F) |
| ATACGGGAGCCAACACCATAGTGTTGGGCCAATACGGTAACGTGTC CTTGGAGAGCAGGTGTGACGGAT |
| SEQ ID NO. 172 (Hydroxylysine 8R) |
| ATCCGTCACACCTGCTCTCCAAGGACACGTTACCGTATTGGCCCAA CACTATGGTGTTGGCTCCCGTAT |
| SEQ ID NO. 173 (Hydroxylysine 9F) |
| ATACGGGAGCCAACACCACCCGTTTTGATCTAATGAGGATACAAT ATTCGTCTAGAGCAGGTGTGACGGAT |
| SEQ ID NO. 174 (Hydroxylysine 9R) |
| ATCCGTCACACCTGCTCTAGACGAATATTGTATCCTCATTAGATCA AAAACGGGTGGTGTTGGCTCCCGTAT |
| SEQ ID NO. 175 (Hydroxylysine 10F) |
| ATACGGGAGCCAACACCAGCTTTTCCTAGAATGATTTCTTTAGCT ACCTGAGAAGAGCAGGTGTGACGGAT |
| SEQ ID NO. 176 (Hydroxylysine 10R) |
| ATCCGTCACACCTGCTCTTCTCAGGTAGCTAAAGAAAATCATTCTA GGAAAAGCTGGTGTTGGCTCCCGTAT |
| SEQ ID NO. 177 (Osteocalcin 2F) |
| ATACGGGAGCCAACACCACGATTAGCAATGAATTATCTACAGAGC AGGTGTGACGGAT |
| SEQ ID NO. 178 (Osteocalcin 2R) |
| ATCCGTCACACCTGCTCTGTAGATAATTCATTGCTAATCGTGGTGTT GGCTCCCGTAT |
| SEQ ID NO. 179 (Osteocalcin 4F) |
| ATACGGGAGCCAACACCAATTCTAACACAGGTTTCTCCGTTTCGTT AGCTGCTAAGAGCAGGTGTGACGGAT |
| SEQ ID NO. 180 (Osteocalcin 4R) |
| ATCCGTCACACCTGCTCTTAGCAGCTAACGAAACGGAGAAACCTGT GTTAGAATTGGTGTTGGCTCCCGTAT |
| SEQ ID NO. 181 (Osteocalcin 7F) |
| ATACGGGAGCCAACACCATTCTATCGTTCCGGACGCTTATGCCTTG CCATCTACAGAGCAGGTGTGACGGAT |
| SEQ ID NO. 182 (Osteocalcin 7R) |
| ATCCGTCACACCTGCTCTGTAGATGGCAAGGCATAAGCGTCCGGAA CGATAGAATGGTGTTGGCTCCCGTAT |
| SEQ ID NO. 183 (Osteocalcin 8F) |
| ATACGGGAGCCAACACCAACTGACTCAGTCTGCTGGTGGGCTATAT TTTTGCGGAGAGCAGGTGTGACGGAT |
| SEQ ID NO. 184 (Osteocalcin 8R) |
| ATCCGTCACACCTGCTCTCCGCAAAAATATAGCCCACCAGCAGACT GAGTCAGTTGGTGTTGGCTCCCGTAT |
| SEQ ID NO. 185 (ProCathepsin K 1F) |
| ATACGGGAGCCAACACCATATAGCCGCGCCTGTGAGTTTTGTGGGA GCAAGAGTAGAGCAGGTGTGACGGAT |
| SEQ ID NO. 186 (ProCathepsin K 1R) |
| ATCCGTCACACCTGCTCTACTCTTGCTCCCACAAAACTCACAGGCG CGGCTATATGGTGTTGGCTCCCGTAT |
| SEQ ID NO. 187 (ProCathepsin K 2F) |
| ATACGGGAGCCAACACCAGCTACAGTGTCAGACGGTTCCACCTTAA CCTCGTCAAGAGCAGGTGTGACGGAT· |
| SEQ ID NO. 188 (ProCathepsin K 2R) |
| ATCCGTCACACCTGCTCTTGACGAGGTTAAGGTGGAACCGTCTGAC ACTGTAGCTGGTGTTGGCTCCCGTAT |
| SEQ ID NO. 189 (ProCathepsin K 3F) |
| ATACGGGAGCCAACACCATTGACTAAGCGATTAGTCCCACAGGTG ACCGGGGAGAGAGCAGGTGTGACGGAT |
| SEQ ID NO. 190 (ProCathepsin K 3R) |
| ATCCGTCACACCTGCTCTCTTCCCGGNCTCCTGTGTGATTAATCTGT TATTCTATGGTGTTGGCTCCCGTAT |
| SEQ ID NO. 191 (ProCathepsin K 4F) |
| ATACGGGAGCCAACACCAATTCTAACACAGGTTTCTCCGTTTCGTT AGCTGCTAAGAGCAGGTGTGACGGAT |
| SEQ ID NO. 192 (ProCathepsin K 4R) |
| ATCCGTCACACCTGCTCTTAGCAGCTAACGAAACGGAGAAACCTGT GTTAGAATTGGTGTTGGCTCCCGTAT |
| SEQ ID NO. 193 (ProCathepsin K 6F) |
| ATACGGGAGCCAACACCACTCATCCCGTTGGAACACTTTAATATGG CCCACTCTAGAGCAGGTGTGACGGAT |
| SEQ ID NO. 194 (ProCathepsin K 6R) |
| ATCCGTCACACCTGCTCTAGAGTGGGCCATATTAAAGTGTTCCAAC GGGATGAGTGGTGTTGGCTCCCGTAT |
| SEQ ID NO. 195 (C-Terminal Telopeptide of Human Collagen (CTx) 1F) |
| ATACGGGAGCCAACACCACTAACTTGTTGCTGATCTTATCCAGAGC AGGTGTGACGGAT |
| SEQ ID NO. 196 (CTx 1R) |
| ATCCGTCACACCTGCTCTGGATAAGATCAGCAACAAGTTAGTGGTG TTGGCTCCCGTAT |
| SEQ ID NO. 197 (CTx 2F) |
| ATACGGGAGCCAACACCACCCGTTTTGATCTAATGAGGATACAAT ATTCGTCTAGAGCAGGTGTGACGGAT |
| SEQ ID NO. 198 (CTx 2R) |
| ATCCGTCACACCTGCTCTAGACGAATATTGTATCCTCATTAGATCA AAAACGGGTGGTGTTGGCTCCCGTAT |
| SEQ ID NO. 199 (CTx 3F) |
| ATACGGGAGCCAACACCAATCGATGGTTAGACTATTACACTAGATG GAATTCATAGAGCAGGTGTGACGGAT |
| SEQ ID NO. 200 (CTx 3R) |
| ATCCGTCACACCTGCTCTATGAATTCCATCTAGTGTAATAGTCTAAC CATCGATTGGTGTTGGCTCCCGTAT |
| SEQ ID NO. 201 (CTx 6F) |
| ATACGGGAGCCAACACCAATCTGCCGACTAGGCCAAGTAATTATAT TCAGCTGGAGAGCAGGTGTGACGGAT |
| SEQ ID NO. 202 (CTx 6R) |
| ATCCGTCACACCTGCTCTCCAGCTGAATATAATTACTTGGCCTAGTC GGCAGATTGGTGTTGGCTCCCGTAT |
| SEQ ID NO. 203 (CTx 7F) |
| ATACGGGAGCCAACACCACAGCTGATATTGGATGGTCCGGCAGAG CAGGTGTGACGGAT |
| SEQ ID NO. 204 (CTx 7R) |
| ATCCGTCACACCTGCTCTGCCGGACCATCCAATATCAGCTGTGGTG TTGGCTCCCGTAT |
| SEQ ID NO. 205 (CTx 8F) |
| ATACGGGAGCCAACACCACAGCTGATATTGGATGGTCCGGCAGAG CAGGTGTGACGGAT |
| SEQ ID NO. 206 (CTx 8R) |
| ATCCGTCACACCTGCTCTGCCGGACCATCCAATATCAGCTGTGGTG TTGGCTCCCGTAT |
| SEQ ID NO. 207 (CTx 11F) |
| ATACGGGAGCCAACACCACATTACAATAGATGTATTGACATATCCG GACAGTCGAGAGCAGGTGTGACGGAT |
| SEQ ID NO. 208 (CTx 11R) |
| ATCCGTCACACCTGCTCTCGACTGTCCGGATATGTCAATACATCTAT TGTAATGTGGTGTTGGCTCCCGTAT |
| SEQ ID NO. 209 (CTx 13F) |
| ATACGGGAGCCAACACCACCCGTTTTTGATCTAATGAGGATACAAT ATTCGTCTAGAGCAGGTGTGACGGAT |
| SEQ ID NO. 210 (CTx 13R) |
| ATCCGTCACACCTGCTCTAGACGAATATTGTATCCTCATTAGATCA AAAACGGGTGGTGTTGGCTCCCGTAT |
| SEQ ID NO. 211 (CTx 14F) |
| ATACGGGAGCCAACACCACTCGTGTAGTGCTGTCTTTGTGGAATCC TTGCATCGAGAGCAGGTGTGACGGAT |
| SEQ ID NO. 212 (CTx 14R) |
| ATCCGTCACACCTGCTCTCGATGCAAGGATTCCACAAAGACAGCAC TACACGAGTGGTGTTGGCTCCCGTAT |
| SEQ ID NO. 213 (CTx 15F) |
| ATACGGGAGCCAACACCACCACGTGACCCATACGATACAACAAAT AATTGCTCAAGAGCAGGTGTGACGGAT |
| SEQ ID NO. 214 (CTx 15R) |
| ATCCGTCACACCTGCTCTTGAGCAATTATTTGTTGTATCGTATGGGT CACGTGGTGGTGTTGGCTCCCGTAT |
| SEQ ID NO. 215 (CTx 16F) |
| ATACGGGAGCCAACACCATCCATAGCTCATCTATACCCTCTTCCGA GTCCCACCAGAGCAGGTGTGACGGAT |
| SEQ ID NO. 216 (CTx 16R) |
| ATCCGTCACACCTGCTCTGGTGGGACTCGGAAGAGGGTATAGATGA GCTATGGATGGTGTTGGCTCCCGTAT |
| SEQ ID NO. 217 (CTx 17F) |
| ATACGGGAGCCAACACCAGACGCGGAACGACTCATCGCAAAATGT CGTGATGCAAGAGCAGGTGTGACGGAT |
| SEQ ID NO. 218 (CTx 17R) |
| ATCCGTCACACCTGCTCTTGCATCACGACATTTTGCGATGAGTCGTT CCGCGTCTGGTGTTGGCTCCCGTAT |
| SEQ ID NO. 219 (CTx 18F) |
| ATACGGGAGCCAACACCATGGTTAGGCTGCTCCATATATTCCCGCC CCGCACGTAGAGCAGGTGTGACGGAT |
| SEQ ID NO. 220 (CTx 18R) |
| ATCCGTCACACCTGCTCTACGTGCGGGGCGGGAATATATGGAGCAG CCTAACCATGGTGTTGGCTCCCGTAT |
| SEQ ID NO. 221 (CTx 19F) |
| ATACGGGAGCCAACACCACCCGTTTTGATCTAATGAGGATACAAT ATTCGTCTAGAGCAGGTGTGACGGAT |
| SEQ ID NO. 222 (CTx 19R) |
| ATCCGTCACACCTGCTCTAGACGAATATTGTATCCTCATTAGATCA AAAACGGGTGGTGTTGGCTCCCGTAT |
| SEQ ID NO. 223 (CTx 20F) |
| ATACGGGAGCCAACACCACCCGTTTTGATCTTATGAGGATACAAT ATTCGTCTAGAGCAGGTGTGACGGAT |
| SEQ ID NO. 224 (CTx 20R) |
| ATCCGTCACACCTGCTCTAGACGAATATTGTATCCTCATAAGATCA AAAACGGGTGGTGTTGGCTCCCGTAT |
| SEQ ID NO. 225 (N-Terminal Telopeptide of Human Collagen (NTx) 2F) |
| ATCCGTCACACCTGCTCTCCGACCAATGTGTGGATCATTACTAATCGACTATTGT GGTGTTGGCTCCCGTAT |
| SEQ ID NO. 226 (NTx 2R) |
| ATACGGGAGCCAACACCACAATAGTCGATTAGTAATGATCCACACATTGGTCGG AGAGCAGGTGTGACGGAT |
| SEQ ID NO. 227 (NTx 4F) |
| ATACGGGAGCCAACACCATAGTTTTGGGCCAATACGGTAACGTGTCCTTGGAGA GCAGGTGTGACGGAT |
| SEQ ID NO. 228 (NTx 4R) |
| ATCCGTCACACCTGCTCTCCAAGGACACGTTACCGTATTGGCCCAAAACTATGGT GTTGGCTCCCGTAT |
| SEQ ID NO. 229 (NTx 8F) |
| ATCCGTCACACCTGCTCTCCAAGGACACGTTACCGTATTGGTCCAGCACTATGGT GTTGGCTCCCGTAT |
| SEQ ID NO. 230 (NTx 8R) |
| ATACGGGAGCCAACACCATAGTGCTGGACCAATACGGTAACGTGTCCTTGGAGA GCAGGTGTGACGGAT |
| SEQ ID NO. 231 (NTx 10F) |
| ATCCGTCACACCTGCTCTAACGTGTGGGTTGAAGTGTCGCCAACAAATTGATAGT GGTGTTGGCTCCCGTAT |
| SEQ ID NO. 232 (NTx 10R) |
| ATACGGGAGCCAACACCACTATCAATTTGTTGGCGACACTTCAACCCACACGTTA GAGCAGGTGTGACGGAT |
| SEQ ID NO. 233 (NTx 11F) |
| ATCCGTCACACCTGCTCTCGACCAAATATTTCCCCCAGCTCTAACCCATGCTGAT GGTGTTGGCTCCCGTAT |
| SEQ ID NO. 234 (NTx 11R) |
| ATACGGGAGCCAACACCATCAGCATGGGTTAGAGCTGGGGGAAATATTTGGTCG AGAGCAGGTGTGACGGAT |
| SEQ ID NO. 235 (NTx 12F) |
| ATACGGGAGCCAACACCATAGTGTTGGGCCAATACGGTAACGTGTCCTTGGAGA GCAGGTGTGACGGAT |
| SEQ ID NO. 236 (NTx 12R) |
| ATCCGTCACACCTGCTCTCCAAGGACACGTTACCGTATTGGCCCAACACTATGGT GTTGGCTCCCGTAT |
| SEQ ID NO. 237 (NTx 13F) |
| ATCCGTCACACCTGCTCTGGGGTCCGCTTGGGAACGATATTCCTGTTGTTTTGTTG GTGTTGGCTCCCGTAT |
| SEQ ID NO. 238 (NTx 13R) |
| ATACGGGAGCCAACACCAACAAAACAACAGGAATATCGTTCCCAAGCGGACCCC AGAGCAGGTGTGACGGAT |
| SEQ ID NO. 239 (NTx 14F) |
| ATCCGTCACACCTGCTCTGATGGCAACATGGGTTAAATCTAACAACACTTTGTAT GGTGTTGGCTCCCGTAT |
| SEQ ID NO. 240 (NTx 14R) |
| ATACGGGAGCCAACACCATACAAAGTGTTGTTAGATTTAACCCATGTTGCCATCA GAGCAGGTGTGACGGAT |
| SEQ ID NO. 241 (NTx 15F) |
| ATACGGGAGCCAACACCAAGGGTGTTCACACTGGCAGGCGACGCCCTCGTGTTG AGAGCAGGTGTGACGGAT |
| SEQ ID NO. 242 (NTx 15R) |
| ATCCGTCACACCTGCTCTCAACACGAGGGCGTCGCCTGCCAGTGTGAACACCCTT GGTGTTGGCTCCCGTAT |
| SEQ ID NO. 243 (25-Hydroxy-Vitamin D₃ (Calcidiol) VD3 1F) |
| ATACGGGAGCCAACACCATAGACAATGGCGTACTTTTCGTAATTCC ACAAGAATAGAGCAGGTGTGACGGAT |
| SEQ ID NO. 244 (VD3 1R) |
| ATCCGTCACACCTGCTCTATTCTTGTGGAATTACGAAAAGTACGCC ATTGTCTATGGTGTTGGCTCCCGTAT |
| SEQ ID NO. 245 (VD3 2F) |
| ATACGGGAGCCAACACCACCACAAAAGCATTCGCCCTTACAGAGC AGGTGTGACGGAT |
| SEQ ID NO. 246 (VD3 2R) |
| ATCCGTCACACCTGCTCTGTAAGGGCGAATGCTTTTGTGGTGGTGTT GGCTCCCGTAT |
| SEQ ID NO. 247 (VD3 3F) |
| ATACGGGAGCCAACACCAGCGTGTAGCTAGTTTCAGGATTGTAGTA TGTAATATAGAGCAGGTGTGACGGAT |
| SEQ ID NO. 248 (VD3 3R) |
| ATCCGTCACACCTGCTCTATATTACATACTACAATCCTGAAACTAG CTACACGCTGGTGTTGGCTCCCGTAT |
| SEQ ID NO. 249 (VD3 5F) |
| ATACGGGAGCCAACACCACGCACATACTAGCTATCTCATCAGAGCA GGTGTGACGGAT |
| SEQ ID NO. 250 (VD3 5R) |
| ATCCGTCACACCTGCTCTGATGAGATAGCTAGTATGTGCGTGGTGT TGGCTCCCGTAT |
| SEQ ID NO. 251 (VD3 6F) |
| ATACGGGAGCCAACACCATCAGAGATCATCTAACGAAAATCATGG GTCTCGCCCAGAGCAGGTGTGACGGAT |
| SEQ ID NO. 252 (VD3 6R) |
| ATCCGTCACACCTGCTCTGGGCGAGACCCATGATTTCGTTAGATG ATCTCTGATGGTGTTGGCTCCCGTAT |
| SEQ ID NO. 253 (VD3 7F) |
| ATACGGGAGCCAACACCAGCAAAGAATAGTGAGCCCTATGATCAT CTGTTCGTCAGAGCAGGTGTGACGGAT |
| SEQ ID NO. 254 (VD3 7R) |
| ATCCGTCACACCTGCTCTGACGAACAGATGATCATAGGGCTCACTA TTCTTTGCTGGTGTTGGCTCCCGTAT |
| SEQ ID NO. 255 (VD3 8F) |
| ATACGGGAGCCAACACCAGACATCATGTCGCATATCTGGATCTAGA GGCTATTCAGAGCAGGTGTGACGGAT |
| SEQ ID NO. 256 (VD3 8R) |
| ATCCGTCACACCTGCTCTGAATAGCCTCTAGATCCAGATATGCGAC ATGATGTCTGGTGTTGGCTCCCGTAT |
| SEQ ID NO. 257 (VD3 10F) |
| ATACGGGAGCCAACACCAGTACGGCGGTGTCCGAACTCACTATACC CAGTTGAAAGAGCAGGTGTGACGGAT |
| SEQ ID NO. 258 (VD3 10R) |
| ATCCGTCACACCTGCTCTTTCAACTGGGTATAGTGAGTTCGGACAC CGCCGTACTGGTGTTGGCTCCCGTAT |
| SEQ ID NO. 259 (VD3 13F) |
| ATACGGGAGCCAACACCAGACCTGACAACGAAAACCCCAGTTGTC GCCATAGCCAGAGCAGGTGTGACGGAT |
| SEQ ID NO. 260 (VD3 13R) |
| ATCCGTCACACCTGCTCTGGCTATGGCGACAACTGGGGTTTTCGTT GTCAGGTCTGGTGTTGGCTCCCGTAT |
| SEQ ID NO. 261 (VD3'14F) |
| ATACGGGAGCCAACACCATAGTGTTGGGCCAATACGGTAACGTGTC CTTGGAGAGCAGGTGTGACGGAT |
| SEQ ID NO. 262 (VD3 14R) |
| ATCCGTCACACCTGCTCTCCAAGGACACGTTACCGTATTGGCCCAA CACTATGGTGTTGGCTCCCGTAT |
| SEQ ID NO. 263 (VD3 15F) |
| ATACGGGAGCCAACACCATAAGCGCAACACAGTCCATCCCTGAGT GAGATAGCGAGAGCAGGTGTGACGGAT |
| SEQ ID NO. 264 (VD3 15R) |
| ATCCGTCACACCTGCTCTCGCTATCTCACTCAGGGATGGACTGTGTT GCGCTTATGGTGTTGGCTCCCGTAT |
| SEQ ID NO. 265 (VD3 16F) |
| ATACGGGAGCCAACACCACGCACATACTAGCTATCTCATCAGAGCA GGTGTGACGGAT |
| SEQ ID NO. 266 (VD3 16R) |
| ATCCGTCACACCTGCTCTGATGAGATAGCTAGTATGTGCGTGGTGT TGGCTCCCGTAT |
| SEQ ID NO. 267 (VD3 17F) |
| ATACGGGAGCCAACACCACTAACTTGTTGCTGATCTTACCAGAGCA GGTGTGACGGAT |
| SEQ ID NO. 268 (VD3 17R) |
| ATCCGTCACACCTGCTCTGGTAAGATCAGCAACAAGTTAGTGGTGT TGGCTCCCGTAT |
| SEQ ID NO. 269 (VD3 18F) |
| ATACGGGAGCCAACACCACCCGTTTTGATCTAATGAGGATACAAT ATTCGTCNAGAGCAGGTGTGACGGAT |
| SEQ ID NO. 270 (VD3 18R) |
| ATCCGTCACACCTGCTCTNGACGAATATTGTATCCTCATTAGATCA AAAACGGGTGGTGTTGGCTCCCGTAT |
| SEQ ID NO. 271 (VD3 19F) |
| ATACGGGAGCCAACACCAGTTGTGGGAACATCAGGCTAAGTATGA GACGGAACGAGAGCAGGTGTGACGGAT |
| SEQ ID NO. 272 (VD3 19R) |
| ATCCGTCACACCTGCTCTCGTTCCGTCTCATACTTAGCCTGATGTTC CCACAACTGGTGTTGGCTCCCGTAT |
| SEQ ID NO. 273 (VD3 20F) |
| ATACGGGAGCCAACACCATAGTGTTGGGCCAATACGGTAACGTGTC CTTGGAGAGCAGGTGTGACGGAT |
| SEQ ID NO. 274 (VD3 20R) |
| ATCCGTCACACCTGCTCTCCAAGGACACGTTACCGTATTGGCCCAA CACTATGGTGTTGGCTCCCGTAT |

| **Botulinum Toxin Type A Aptamer Sequences** |
|---|
| SEQ ID NO. 275 (Botulinum Toxin Type A-Light Chain (BoNT A-LC1)) |
| CATCCGTCACACCTGCTCTGGGGATGTGTGGTGTTGGCTCCCGTATCAAGGGCGA ATTCT |
| SEQ ID NO. 276 (BoNT A-LC2) |
| GTAGGCAGTGTGGACGAGACCCCTACACACCACAACCGAGGGCATAGTTCCCGC TTAAGA |
| SEQ ID NO.277 (Botulinum Toxin Type A-Holotoxin (BoNT A-HT1)) |
| CATCCGTCACACCTGCTCTGCTATCACATGCCTGCTGAAGTGGTGTTGGCTCCCGT ATCA |
| SEQ ID NO. 278 (BoNT A-HT2) |
| GTAGGCAGTGTGGACGAGACGATAGTGTACGGACGACTTCACCACAACCGAGGG CATAGT |

| ***E. coli* Outer Membrane Proteins (OMPs)** |
|---|
| SEQ ID NO. 279 (EcO - 1F) |
| ATCCGTCACACCTGCTCTCGATGTCTGGGCCCTAATATTGGTTTGCTTGTACCA TGGTGTTGGCTCCCGTAT |
| SEQ ID NO. 280 (EcO - 1R) |
| ATACGGGAGCCAACACCATGGTACAAGCAAACCAATATTAGGGCCCAGACATCG AGAGCAGGTGTGACGGAT |
| SEQ ID NO. 281 (EcO - 2F) |
| ATACGGGAGCCAACACCATGATACCCTAAGGTAGGGGAGGCCTAAGCGCCACGT AGAGCAGGTGTGACGGAT |
| SEQ ID NO. 282 (EcO - 2R) |
| ATCCGTCACACCTGCTCTACGTGGCGCTTAGGCCTCCCCTACCTTAGGGTATCA TGGTGTTGGCTCCCGTAT |
| SEQ ID NO. 283 (EcO - 3F) |
| ATACGGGAGCCAACACCACGCATCCCCCGCCGGGCCCGCGCCCCGCTCGCAGAC AGAGCAGGTGTGACGGAT |
| SEQ ID NO. 284 (EcO - 3R) |
| ATCCGTCACACCTGCTCTGTCTGCGAGCGGGGCGCGGCCCGGCGGGGGATGCG TGGTGTTGGCTCCCGTAT |
| SEQ ID NO. 285 (EcO - 4F (73)) |
| ATCCGTCACACCTGCTCTACGGCGCTCCCAACAGGCCTCTCCTTACGGCATATTA TGGTGTTGGCTCCCGTAT |
| SEQ ID NO. 286 (EcO - 4R (73)) |
| ATACGGGAGCCAACACCATAATATGCCGTAAGGAGAGGCCTGTTGGGAGCGCCG T AGAGCAGGTGTGACGGAT |
| SEQ ID NO. 287 (EcO-5F) |
| ATACGGGAGCCAACACCAGGAAAAAAAGAGCCTGTGAAGATTGTAATATCAGTT AGAGCAGGTGTGACGGAT |
| SEQ ID NO. 288 (EcO - 5R) |
| ATCCGTCACACCTGCTCTAACTGATATTACAATCTTCACAGGCTCTTTTTTTCC TGGTGTTGGCTCCCGTAT |
| SEQ ID NO. 289 (EcO - 7Fa) |
| ATCCGTCACACCTGCTCTCGGAGGTAGACTAGGATTGCGGCGGGGGTCAGGTA TGGTGTTGGCTCCCGTAT |
| SEQ ID NO. 290 (EcO - 7Fb) |
| ATACGGGAGCCAACACCACAAAAGCCTTACCTAACTGCCAACAATGAATAGCA AGAGCAGGTGTGACGGAT |
| SEQ ID NO. 291 (EcO - 7Ra) |
| ATCCGTCACACCTGCTCTTGCTATTCATTGTTGGCAGTTAGGTAAGGCTTTTGT TGGTGTTGGCTCCCGTAT |
| SEQ ID NO. 292 (EcO - 7Rb) |
| ATACGGGAGCCAACACCATACCTGACCCCCCGCCGCAATCCTAGTCTACCTCCG AGAGCAGGTGTGACGGAT |
| SEQ ID NO. 293 (EcO - 8F) |
| ATACGGGAGCCAACACCACGACTAACACGACCGTTGGGGGGGCTCGCGCGGGC AGAGCAGGTGTGACGGAT |
| SEQ ID NO. 294 (EcO - 8R) |
| ATCCGTCACACCTGCTCTGCCCGCGCGAGCCCCCCCAACGGTCGTGTTAGTCG TGGTGTTGGCTCCCGTAT |
| SEQ ID NO. 295 (EcO - 9F) |
| ATACGGGAGCCAACACCAGTCCCCGCCCAGCCGTGAGCCGTACCCCCGCACACC AGAGCAGGTGTGACGGAT |
| SEQ ID NO. 296 (EcO - 9R) |
| ATCCGTCACACCTGCTCTGGTGTGCGGGGGTACGGCTCACGGCTGGGCGGGGAC TGGTGTTGGCTCCCGTAT |
| SEQ ID NO. 297 (EcO - 10F) |
| ATCCGTCACACCTGCTCTCAAGGTTGGGCCTGCAAGAGCAAAAACGGGGCGGGA TGGTGTTGGCTCCCGTAT |
| SEQ ID NO. 298 (EcO - 10R) |
| ATACGGGAGCCAACACCATCCCGCCCCGTTTTTGCTCTTGCAGGCCCAACCTTG AGAGCAGGTGTGACGGAT |
| SEQ ID NO. 299 (EcO - 11F) |
| ATCCGTCACACCTGCTCTACTTGGCTTGCGACTATTATTCACAGGGCCAAAGAC TGGTGTTGGCTCCCGTAT |
| SEQ ID NO. 300 (EcO - 11R) |
| ATACGGGAGCCAACACCAGTCTTTGGCCCTGTGAATAATAGTCGCAAGCCAAGT AGAGCAGGTGTGACGGAT |
| SEQ ID NO. 301 (EcO - 12F (69)) |
| ATACGGGAGCCAACACCATAGTGTTGGACCAATACGGTAACGTGTCCTTGG AGAGCAGGTGTGACGGAT |
| SEQ ID NO. 302 (EcO - 12R (69)) |
| ATCCGTCACACCTGCTCTCCAAGGACACGTTACCGTATTGGTCCAACACTA TGGTGTTGGCTCCCGTAT |
| SEQ ID NO. 303 (EcO - 17F) |
| ATCCGTCACACCTGCTCTTGGAATGTCGGTGTTTTTCCAATTCCTTGGGTCGTG TGGTGTTGGCTCCCGTAT |
| SEQ ID NO. 304 (EcO - 17R) |
| ATACGGGAGCCAACACCA CACGACCCAAGGAATTGGAAAAACACCGACATTCCA AGAGCAGGTGTGACGGAT |
| SEQ ID NO. 305 (EcO - 18F) |
| ATCCGTCACACCTGCTCTGCGACGGCGACGCGGTCCGGGCGGGGGTGGAGGACG TGGTGTTGGCTCCCGTAT |
| SEQ ID NO. 306 (EcO - 18R) |
| ATACGGGAGCCAACACCACGTCCTCCACCCCCGCCGGACCGCGTCGCCGTCGC AGAGCAGGTGTGACGGAT |
| SEQ ID NO. 307 (EcO - 19Fa) |
| ATACGGGAGCCAACACCAGAGGGTTCTAGGGTCACTTCCATGAGAATGGCTCAC AGAGCAGGTGTGACGGAT |
| SEQ ID NO. 308 (EcO - 19Fb) |
| ATCCGTCACACCTGCTCTGGCCTGGGGACGCGAGGGAGGCGGGGGGAGTCGTGG TGGTGTTGGCTCCCGTAT |
| SEQ ID NO. 309 (EcO - 19Ra) |
| ATACGGGAGCCAACACCACCACGACTCCCCCGCCTCCCTCGCGTCCCCAGGCCA GAGCAGGTGTGACGGAT |
| SEQ ID NO. 310 (EcO - 19Rb) |
| ATCCGTCACACCTGCTCT GTGAGCCATTCTCATGGAAGTGACCCTAGAACCCTC TGGTGTTGGCTCCCGTAT |
| SEQ ID NO. 311 (EcO - 20F) |
| ATCCGTCACACCTGCTCTCACAGGGCCTCTTACTATACAGTTCTCCAGCGCTGC TGGTGTTGGCTCCCGTAT |
| SEQ ID NO. 312 (EcO - 20R) |
| ATACGGGAGCCAACACCAGCAGCGCTGGAGAACTGTATAGTAAGAGGCCCTGTG AGAGCAGGTGTGACGGAT |
| SEQ ID NO. 313 (EcO - 21F) |
| ATCCGTCACACCTGCTCTGCACGGGCTCAGTTTGGCTTTGTATCCTAAGAGAGA TGGTGTTGGCTCCCGTAT |
| SEQ ID NO. 314 (EcO - 21R) |
| ATACGGGAGCCAACACCATCTCTCTTAGGATACAAAGCCAAACTGAGCCCGTGC AGAGCAGGTGTGACGGAT |
| SEQ ID NO. 315 (EcO - 22F) |
| ATACGGGAGCCAACACCAGGGGTGGCGAACATGGTATAACTTGATAAGTGTGA AGAGCAGGTGTGACGGAT |
| SEQ ID NO. 316 (EcO - 22R) |
| ATCCGTCACACCTGCTCTTCACACTTATCAAGTTATACCATGTTCGCCACCCCC TGGTGTTGGCTCCCGTAT |
| SEQ ID NO. 317 (EcO - 23F) |
| ATACGGGAGCCAACACCACTCCGACACCGGCCGCCGGCACCACCCACTCCCCCT AGAGCAGGTGTGACGGAT |
| SEQ ID NO. 318 (EcO - 23R) |
| ATCCGTCACACCTGCTCTAGGGGGAGTGGGTGGTGCCGGCGGCCGGTGTCGGAG TGGTGTTGGCTCCGTAT |
| SEQ ID NO. 319 (EcO - 24F) |
| ATACGGGAGCCAACACCATCCGGCGCGCCCTCCTCCCCCACTGCTCCCCGCCCG AGAGCAGGTGTGACGGAT |
| SEQ ID NO. 320 (EcO - 24R) |
| ATCCGTCACACCTGCTCTCGGGCGGGGAGCAGTGGGGGAGGAGGGCGCGCCGGA TGGTGTTGGCTCCCGTAT |
| SEQ ID NO. 321 (EcO - 25F) |
| ATACGGGAGCCAACACCATACGCAGAGGTCCCCTACCCAGGCCAGCCGGATGCC AGAGCAGGTGTGACGGAT |
| SEQ ID NO. 322 (EcO - 25R) |
| ATCCGTCACACCTGCTCTGGCATCCGGCTGGCCTGGGTAGGGGACCTCTGCGTA TGGTGTTGGCTCCCGTAT |

| **Shipa toxins (Shiga-like Toxin type 1; Stx-1)** |
|---|
| SEQ ID NO. 323 (SH - 2F) |
| ATCCGTCACACCTGCTCTGGAGACATTAAAAACCGGAGTTTATTTATACCTTTCT GGTGTTGGCTCCCGTAT |
| SEQ ID NO. 324 (SH - 2R) |
| ATACGGGAGCCAACACCAGAAAGGTATAAATAAACTCCGGTTTTTAATGTCTCCA GAGCAGGTGTGACGGAT |
| SEQ ID NO. 325 (SH - 3F (59)) |
| ATACGGGAGCCAACACCACTAACTTGTTGCTGATCTTATCCAGAGCAGGTGTGAC GGAT |
| SEQ ID NO. 326 (SH - 3R (59)) |
| ATCCGTCACACCTGCTCTGGATAAGATCAGCAACAAGTTAGTGGTGTTGGCTCCC GTAT |
| SEQ ID NO. 327 (SH - 4F (58)) |
| ATCCGTCACACCTGCTCTGCATGGAGAGTTTTTGGTCAGTGGTGTTGGCTCCCGT AT |
| SEQ ID NO. 328 (SH - 4R (58)) |
| ATACGGGAGCCAACACCACTGACCAAAAAACTCTCCATGCAGAGCAGGTGTGAC GGAT |
| SEQ ID NO. 329 (SH - 6F (58)) |
| ATACGGGAGCCAACACCACGTTAACGCGTAGCCTTTGGACAGAGCAGGTGTGAC GGAT |
| SEQ ID NO. 330 (SH - 6R (58)) |
| ATCCGTCACACCTGCTCTGTCCAAAGGCTACGCGTTAACGTGGTGTTGGCTCCCG TAT |
| SEQ ID NO. 331 (SH - 8/21/23/24/25F (59)) |
| ATCCGTCACACCTGCTCTGCCGGACCATCCAATATCAGCTGTGGTGTTGGCTCCC GTAT |
| SEQ ID NO. 332 (SH - 8/21/23/24/25 Rev (59)) |
| ATACGGGAGCCAACACCACAGCTGATATTGGATGGTCCGGCAGAGCAGGTGTGA CGGAT |
| SEQ ID NO. 333 (SH - 9F) |
| ATCCGTCACACCTGCTCTCGTCCGTCATTAAGTTCGGAGGCTGGCGGGTTGCGTT GGTGTTGGCTCCCGTAT |
| SEQ ID NO. 334 (SH - 9R) |
| ATACGGGAGCCAACACCAACGCAACCCGCCAGCCTCCGAACTTAATGACGGACG AGAGCAGGTGTGACGGAT |
| SEQ ID NO. 335 (SH - 10F) |
| ATACGGGAGCCAACACCATTCTATCGTTCCGGACGCTTATGCCTTGCCATCTACA GAGCAGGTGTGACGGAT |
| SEQ ID NO. 336 (SH - 10R) |
| ATCCGTCACACCTGCTCTGTAGATGGCAAGGCATAAGCGTCCGGAACGATAGAA TGGTGTTGGCTCCCGTAT |
| SEQ ID NO. 337 (SH - 11F) |
| TCCGTCACACCTGCTCTAACTCTTACTACTTTGTTGCTATCACATTCAACTG TTGGTGTTGGCTCCCGTAT |
| SEQ ID NO. 338 (SH- 11R) |
| ATACGGGAGCCAACACCAACAGTTGAATGTGATAGCAACAAAGTAGTAAGAGTT AGAGCAGGTGTGACGGAT |
| SEQ ID NO. 339 (SH - 12 F(58)) |
| ATCCGTCACACCTGCTCTGGCCTTTCACCAAGCGTCCTTGTGGTGTTGGCTCCCGT AT |
| SEQ ID NO. 340 (SH - 12R (58)) |
| ATACGGGAGCCAACACCACAAGGACGCTTGGTGAAAGGCCAGAGCAGGTGTGAC GGAT |
| SEQ ID NO. 341 (SH - 16F (58)) |
| ATCCGTCACACCTGCTCTGGCACCGAGCACGGAACCCAGTGGTGTTGGCTCCCG TAT |
| SEQ ID NO. 342 (SH - 16R (58)) |
| ATACGGGAGCCAACACCACTGGGTTCCCGTGCTCGGTGCCAGAGCAGGTGTGAC GGAT |
| SEQ ID NO. 343 (SH - 17F (69)) |
| ATACGGGAGCCAACACCATAGTGTTGGGCCAATACGGTAACGTGTCCTTGGAGA GCAGGTGTGACGGAT |
| SEQ ID NO. 344 (SH - 17R (69)) |
| ATCCGTCACACCTGCTCTCCAAGGACACGTTACCGTATTGGCCCAACACTATGGT GTTGGCTCCCGTAT |
| SEQ ID NO. 345 (SH -18F) |
| ATCCGTCACACCTGCTCTACCCGATGCCGCCCCGGGATTGTTGTATGACCATCTT GGTGTTGGCTCCCGTAT |
| SEQ ID NO. 346 (SH - 18R) |
| ATACGGGAGCCAACACCAAGATGGTCATACAACAATCCCGGGGCGGCATCGGGT AGAGCAGGTGTGACGGAT |
| SEQ ID NO. 347 (SH - 19F) |
| ATACGGGAGCCAACACCACCCCATGAGTACACGTGAACGGACACAGCCTCCGGC AGAGCAGGTGTGACGGAT |
| SEQ ID NO. 348 (SH - 19R) |
| ATCCGTCACACCTGCTCTGCCGGAGGCTGTGTCCGTTCACGTGTACTCATGGGGT GGTGTTGGCTCCCGTAT |
| SEQ ID NO. 349 (SH - 20F) |
| ATCCGTCACACCTGCTCTTAACCATTCATTTCTTTTGTGGTATGACCGTTCGCCTG .        GTGTTGGCTCCCGTAT |
| SEQ ID NO. 350 (SH - 20R) |
| ATACGGGAGCCAACACCAGGCGAACGGTCATACCACAAAAGAAATGAATGGTTA AGAGCAGGTGTGACGGAT |
| SEQ ID NO. 351 (SH - 22F (58)) |
| ATCCGTCACACCTGCTCTGGGGCTCTTTCGTTAACCAGGTGGTGTTGGCTCCCGT AT |
| SEQ ID NO. 352 (SH - 22R (58)) |
| ATACGGGAGCCAACACCACCTGGTTAACGAAAAGAGCCCCAGAGCAGGTGTGAC GGAT |

| ***S. typhimurium* (*S. enterica* serovar Typhimurium type 13311) OMPs** |
|---|
| SEQ ID NO. 353 (StO - 2F) |
| ATACGGGAGCCAACACCAGATAAATTTTGCGTTCATTCTTATTTCCT GTCCGCCAGAGCAGGTGTGACGGAT |
| SEQ ID NO. 354 (StO - 2R) |
| ATCCGTCACACCTGCTCTGGCGGACAGGAAATAAGAATGAACGCA AAATTTATCTGGTGTTGGCTCCCGTAT |
| SEQ ID NO. 355 (StO - 4F) |
| ATACGGGAGCCAACACCAGATAAATTTTGGTTCATTCTTATTTCCTG TCCGCCAGAGCAGGTGTGACGGAT (71) |
| SEQ ID NO. 356 (StO - 4R) |
| ATCCGTCACACCTGCTCTGGCGGACAGGAAATAAGAATGAACCAA AATTTATCTGGTGTTGGCTCCCGTAT (71) |
| SEQ ID NO. 357 (StO - 5F) |
| ATACGGGAGCCAACACCACGGGGCTACCAGCACCGTCACCCCTCAT TCTGCCACAGAGCAGGTGTGACGGAT |
| SEQ ID NO. 358 (StO - 5R) |
| ATCCGTCACACCTGCTCTGTGGCAGAATGAGGGGTGACGGTGCTGG TAGCCCCGTGGTGTTGGCTCCCGTAT |
| SEQ ID NO. 359 (StO - 6F) |
| ATACGGGAGCCAACACCAAAAGATGGAAAACACTGGAAGGAAAAT GCGGTCAGAGCAGGTGTGACGGAT (69) |
| SEQ ID NO. 360 (StO - 6R) |
| ATCCGTCACACCTGCTCTGACCGCATTTTCCTTCCAGTGTTTTCCAT CTTTTGGTGTTGGCTCCCGTAT (69) |
| SEQ ID NO. 361 (StO - 7F) |
| ATACGGGAGCCAACACCACCGGGCCGATGGGCACCAGGAACTCTC GGACGAGTGAGAGCAGGTGTGACGGAT |
| SEQ ID NO. 362 (StO - 7R) |
| ATCCGTCACACCTGCTCTCACTCGTCCGAGAGTTCCTGGTGCCCATC GGCCCGGTGGTGTTGGCTCCCGTAT |
| SEQ ID NO. 363 (StO - 8F) |
| ATACGGGAGCCAACACCACAGCTGATATTGGATGGTCCGGCAGAG CAGGTGTGACGGAT (59) |
| SEQ ID NO. 364 (StO - 8R) |
| ATCCGTCACACCTGCTCTGCCGGACCATCCAATATCAGCTGTGGTG TTGGCTCCCGTAT (59) |
| SEQ ID NO. 365 (StO - 9F) |
| ATACGGGAGCCAACACCAGTCGAAAGGCGGCCGTCCAGTCGAGTG ATTTGACCTAGAGCAGGTGTGACGGAT |
| SEQ ID NO. 366 (StO - 9R) |
| ATCCGTCACACCTGCTCTAGGTCAAATCACTCGACTGGACGGCCGC CTTTCGACTGGTGTTGGCTCCCGTAT |
| SEQ ID NO. 367 (StO - 10F) |
| ATACGGGAGCCAACACCACGGGGCGTGCCGTCAAAAGACCGAGAT GTGGCTGCGAGAGCAGGTGTGACGGAT |
| SEQ ID NO. 368 (StO - 10R) |
| ATCCGTCACACCTGCTCTCGCAGCCACATCTCGGTCTTTTGACGGCA CGCCCCGTGGTGTTGGCTCCCGTAT |
| SEQ ID NO. 369 (StO - 11/13F) |
| ATACGGGAGCCAACACCACTAACTTGTTGCTGATCTTATCCAGAGC AGGTGTGACGGAT (59) |
| SEQ ID NO. 370 (StO - 11/13R) |
| ATCCGTCACACCTGCTCTGGATAAGATCAGCAACAAGTTAGTGGTG TTGGCTCCCGTAT (59) |
| SEQ ID NO. 371 (StO - 12F) |
| ATACGGGAGCCAACACCATTTAGCGTAGGGCTCGCTTATCATTTCT CATTCCCTAGAGCAGGTGTGACGGAT |
| SEQ ID NO. 372 (StO - 12R) |
| ATCCGTCACACCTGCTCTAGGGAATGAGAAATGATAAGCGAGCCCT ACGCTAAATGGTGTTGGCTCCCGTAT |
| SEQ ID NO. 373 (StO - 14F) |
| ATACGGGAGCCAACACCACCGCAACCCAAATCTCTACACGGATTAT CGTCGAGCAGAGCAGGTGTGACGGAT |
| SEQ ID NO. 374 (StO - 14R) |
| ATCCGTCACACCTGCTCTGCTCGACGATAATCCGTGTAGAGATTTG GGTTGCGGTGGTGTTGGCTCCCGTAT |
| SEQ ID NO. 375 (StO - 16F) |
| ATACGGGAGCCAACACCAACACATTCTATAATGAATGTTCCTGTCG CGTTGCGTAGAGCAGGTGTGACGGAT |
| SEQ ID NO. 376 (StO - 16R) |
| ATCCGTCACACCTGCTCTACGCAACGCGACAGGAACATTCATTATA GAATGTGTTGGTGTTGGCTCCCGTAT |
| SEQ ID NO. 377 (StO - 17F) |
| ATACGGGAGCCAACACCAGCCTACCCCCCCTGTACGAGGGCCGCA ACCACGTAGAGAGCAGGTGTGACGGAT |
| SEQ ID NO. 378 (StO - 17R) |
| ATCCGTCACACCTGCTCTCTACGTGGTTGCGGCCCTCGTACAGGGG GGGTAGGCTGGTGTTGGCTCCCGTAT |
| SEQ ID NO. 379 (StO - 18F) |
| ATACGGAGCCAACACCACATCTAGCACGAGACCCTATCCCAGAG CAGGTGTGACGGAT (59) |
| SEQ ID NO. 380 (StO - 18R) |
| ATCCGTCACACCTGCTCTGGGATAGGGTCTCGTGCTAGATGTGGTG TTGGCTCCCGTAT (59) |
| SEQ ID NO. 381 (StO - 19F) |
| ATACGGGAGCCAACACCAACAGCGACTCGAGTCTGACGACTCGCG GGGCAAATGAGAGCAGGTGTGACGGAT |
| SEQ ID NO. 382 (StO - 19R) |
| ATCCGTCACACCTGCTCTCATTTGCCCCGCGAGTCGTCAGACTCGA GTCGCTGTTGGTGTTGGCTCCCGTAT |
| SEQ ID NO. 383 (StO - 20/24F) |
| ATACGGGAGCCAACACCATAGTGTTGGGCCAATACGGTAACGTGTC CTTGGAGAGCAGGTGTGACGGAT (69) |
| SEQ ID NO. 384 (StO - 20/24R) |
| ATCCGTCACACCTGCTCTCCAAGGACACGTTACCGTATTGGCCCAA CACTATGGTGTTGGCTCCCGTAT (69) |
| SEQ ID NO. 385 (StO - 21F) |
| ATACGGGAGCCAACACCACTAAGGAGAGGTCGCGACAGACTCTTC TGGTCAAGGAGAGCAGGTGTGACGGAT |
| SEQ ID NO. 386 (StO - 21R) |
| ATCCGTCACACCTGCTCTCCTTGACCAGAAGAGTCTGTCGCGACCT CTCCTTAGTGGTGTTGGCTCCCGTATG |
| SEQ ID NO. 387 (StO - 22F) |
| ATACGGGAGCCAACACCAACTTCGACTCAAAGAAGTCCACGTGAG ACTGGTGGAAGAGCAGGTGTGACGGAT |
| SEQ ID NO. 388 (StO - 22R) |
| ATCCGTCACACCTGCTCTTCCACCAGTCTCACGTGGACTTCTTTGAG TCGAAGTTGGTGTTGGCTCCCGTAT |
| SEQ ID NO. 389 (StO - 23F) |
| ATACGGAGCCAACACCACCCGGGGAGACCCGCACGGGCGCACAA TCCTTGTCGAGAGCAGGTGTGACGGAT |
| SEQ ID NO. 390 (StO - 23R) |
| ATCCGTCACACCTGCTCTCGACAAGGATTGTGCGCCCGTGCGGGTC TCCCCGGGTGGTGTTGGCTCCCGTAT |
| SEQ ID NO. 391 (StO - 25F) |
| ATACGGGAGCCAACACCAGCTGGACCAAACTACGCCCATTGTGGG GGTCCCCGGAGAGCAGGTGTGACGGAT |
| SEQ ID NO. 392 (StO - 25R) |
| ATCCGTCACACCTGCTCTCCGGGGACCCCCACAATGGGCGTAGTTTGGTCCAGCT GGTGTTGGCTCCCGTAT |

| **Acetylcholine (ACh)** |
|---|
| SEQ ID NO. 393 (ACh1aF) |
| ATACGGGAGCCAACACCACGATACCCGCTTATGAATTTTAAATTAATTGTGATCA GAGCAGGTGTGACGGAT |
| SEQ ID NO. 394 (ACh 1aR) |
| ATCCGTCACACCTGCTCTGATCACAATTAATTTAAAATTCATAAGCGGGTATCGT GGTGTTGGCTCCCGTAT |
| SEQ ID NO. 395 (ACh 1bF) |
| ATACGGGAGCCAACACCAACTTTCACACATACTTGTTATACCACACGATCTTTTA GAGCAGGTGTGACGGAT |
| SEQ ID NO. 396 (ACh 1bR) |
| ATCCGTCACACCTGCTCTAAAAGATCGTGTGGTATAACAAGTATGTGTGAAAGTT GGTGTTGGCTCCCGTAT |
| SEQ ID NO. 397 (ACh 2F) |
| ATACGGGAGCCAACACCACTTTGTAACTCATTTGTAGTTTGGGTTGCTCCCCCTA GAGCAGGTGTGACGGAT |
| SEQ ID NO. 398 (ACh 2R) |
| ATCCGTCACACCTGCTCTAGGGGGAGCAACCCAAACTACAAATGAGTTACAAAG TGGTGTTGGCTCCCGTAT |
| SEQ ID NO. 399 (ACh 3F) |
| ATACGGGAGCCAACACCATTTCCCGCTTATCTTCATCCACTGCTTAGCATATGTA GAGCAGGTGTGACGGAT |
| SEQ ID NO. 400 (ACh 3R) |
| ATCCGTCACACCTGCTCTACATATGCTAAGCAGTGGATGAAGATAAGCGGGAAA TGGTGTTGGCTCCCGTAT |
| SEQ ID NO. 401 (ACh 5F) |
| ATACGGGAGCCAACACCAGGCACTGTATCACACCGTCAAGAATGTGATCCCCTG AGAGCAGGTGTGACGGAT |
| SEQ ID NO. 402 (ACh 5R) |
| ATCCGTCACACCTGCTCTCAGGGGATCACATTCTTGACGGTGTGATACAGTGCCT GGTGTTGGCTCCCGTAT |
| SEQ ID NO. 403 (ACh 6F) |
| ATACGGGAGCCAACACCATGTCATTTACCTTCATCATGACAGTGTTAGTATACGA GAGCAGGTGTGACGGAT |
| SEQ ID NO. 404 (ACh 6R) |
| ATCCGTCACACCTGCTCTAGGGGATCAAAGCTATGCGACCATGCGAGTGG ATACTGGTGTTGGCTCCCGTAT |
| SEQ ID NO. 405 (ACh 7F) |
| ATACGGGAGCCAACACCAGTTGCCGCCTACCTTGATTATTCTACATTACCCGTTA GAGCAGGTGTGACGGAT |
| SEQ ID NO. 406 (ACh 7R) |
| ATCCGTCACACCTGCTCTAACGGGTAATGTAGAATAATCAAGGTAGGCGGCAAC TGGTGTTGGCTCCCGTAT |
| SEQ ID NO. 407 (ACh 8F) |
| ATACGGGAGCCAACACCAGTATACATACGAAGAGTTGAAACCAATGCTTCGTTC AGAGCAGGTGTGACGGAT |
| SEQ ID NO. 408 (ACh 8R) |
| ATCCGTCACACCTGCTCTGAACGAAGCATTGGTTTCAACTCTTCGTATGTATACTG GTGTTGGCTCCCGTAT |
| SEQ ID NO. 409 (ACh 9F) |
| ATACGGGAGCCAACACCATACCCCGAATGGCTGTTTTCAGTACCAATATGACTCA GAGCAGGTGTGACGGAT |
| SEQ ID NO. 410 (ACh 9R) |
| ATCCGTCACACCTGCTCTGAGTCATATTGGTACTGAAAACAGCCATTCGGGGTAT GGTGTTGGCTCCCGTAT |
| SEQ ID NO. 411 (ACh 10F) |
| ATACGGGAGCCAACACCACTGTCACGATCGTCGTTCCTTTTAATCCTTGTGTCTA GAGCAGGTGTGACGGAT |
| SEQ ID NO. 412 (ACh 10R) |
| ATCCGTCACACCTGCTCTAGACACAAGGATTAAAAGGAACGACGATCGTGACAG TGGTGTTGGCTCCCGTAT |
| SEQ ID NO. 413 (ACh 11F) |
| ATACGGGAGCCAACACCACTGGACACTGACCCTCGCACTAGCTTTCTGACGGGTA GAGCAGGTGTGACGGAT |
| SEQ ID NO. 414 (ACh 11R) |
| ATCCGTCACACCTGCTCTACCCGGCCGAAGAATAGTGCTCGGTACTTAGTCGCGT GGTGTTGGCTCCCGTAT |
| SEQ ID NO. 415 (ACh 12F) |
| ATACGGGAGCCAACACCATTTGGACTTTAAATAGTGGACTCCTTCTTTGTCTCGA GAGCAGGTGTGACGGAT |
| SEQ ID NO. 416 (ACh 12R) |
| ATCCGTCACACCTGCTCTCGAGACAAAGAAGGAGTCCACTATTTAAAGTCCAAAT GGTGTTGGCTCCCGTAT |

| **Gram Negative Quorum Sensing Molecules (N-Acylhomoserine Lactones; AHLs)** |
|---|
| SEQ ID NO. 417 (Dec AHL 1F) |
| ATACGGGAGCCAACACCATCCTAACTGGTCTAATTTTGCTGTTACCGATCCCGA GAGCAGGTGTGACGGAT |
| SEQ ID NO. 418 (Dec AHL 1R) |
| ATCCGTCACTCCTGCTCTCGGGATCGGTAACAGCAAAAATTAGACCAGTTAGGAT GGTGTTGGCTCCCGTAT |
| SEQ ID NO. 419 (Dec AHL 13F) |
| ATACGGGAGCCAACACCAGCCTGACGAAAAAATTTTATCACTAAGTGATACGCA AGAGCAGGTGTGACGGAT |
| SEQ ID NO. 420 (Dec AHL 13R) |
| ATCCGTCACACCTGCTCTTGCGTATCACTTAGTGATAAAATTTTTCGTCAGGCTG GTGTTGGCTCCCGTAT |
| SEQ ID NO. 421 (Dec AHL 14F) |
| ATACGGGAGCCAACACCAGACCTACTTCAGAAACGGAAATGTTCTTAGCCGTCA GAGCAGGTGTGACGGAT |
| SEQ ID NO. 422 (Dec AHL 14R) |
| ATCCGTCACACCTGCTCTGACGGCTAAGAACATTTCCGTTTCTGAAGTAGGTCTG GTGTTGGCTCCCGTAT |
| SEQ ID NO. 423 (Dec AHL 15F) |
| ATACGGGAGCCAACACCAGGCCAACGAAACTCCTACTACATATAATGCTTATGC AGAGCAGGTGTGACGGAT |
| SEQ ID NO. 424 (Dec AHL 15R) |
| ATCCGTCACACCTGCTCTGCATAAGCATTATATGTAGTAGGAGTTTCGTTGGCCT GGTGTTGGCTCCCGTAT |
| SEQ ID NO. 425 (Dec AHL 17F) |
| ATACGGGAGCCAACACCATCCTAACTGGTCTAATTTTTGCTGTTACCGATCCCGA GAGCAGGTGTGACGGAT |
| SEQ ID NO. 426 (Dec AHL 17R) |
| ATCCGTCACACCTGCTCTCGGGATCGGTAACAGCAAAAATTAGACCAGTTAGGAT GGTGTTGGCTCCCGTAT |

## Claims

1. A method of a sandwich assay, run by producing and assembling DNA or RNA aptamer-magnetic bead conjugates, for the capture and detection of a target analyte in a bulk solution, comprising:
combining said bulk solution, an aptamer-magnetic bead conjugate ("aptamer-MB"), and an aptamer-fluorophore conjugate in a cuvette, wherein said aptamer-MB is able to bind with said target analyte at a first binding site and said aptamer-fluorophore conjugate is able to bind with said target analyte at a second binding site to form an analyte-aptamer-fluorophore complex, and wherein said cuvette has a translucent surface area so as to enable a fluorescent assay;
allowing said aptamer-MB to bind with said target analyte at said first binding site and said aptamer-fluorophore conjugate to bind with said target analyte at said second binding site to form said analyte-aptamer-fluorophore complex;
**characterised in that** the method further comprises
positioning said cuvette in a position such that said translucent surface area is oriented substantially vertically;
adhering said analyte-aptamer-fluorophore complex to said cuvette translucent surface area by applying an external magnetic field to attract said magnetic bead; and
removing the external magnet; and
assaying said analyte-aptamer-fluorophore complex that is adhered to said cuvette translucent surface area by illuminating the solution from the opposite side of the cuvette by an excitation source and placing the cuvette side with adherent assay materials proximal to a photodetector.

2. The method of Claim 1 wherein said bulk solution is acidic.

3. The method of Claim 1 or 2 wherein said method does not include a wash step.

4. The method of Claim 1 or 2 wherein said analyte-aptamer-fluorophore complex is effectively partitioned away from said bulk solution to enhance detectability.

5. The method of Claim 1 or 2 wherein said cuvette is made from polystyrene, clear plastic, or glass.

6. The method of Claim 5 in which said cuvette translucent surface area, on which said analyte-aptamer-fluorophore complex adheres, is formed as a square, rectangular, round, oval, or flat container, vial, tube, cylinder, cassette, or cartridge.

7. The method of Claim 1 or 2, wherein said aptamer-MB and said aptamer-fluorophore will not bind, base pair, or hybridize with each other in said bulk solution.

8. The method of Claim 1 or 2, wherein said fluorophore in said aptamer-fluorophore conjugate is a quantum dot ("QD"), fluorescent or phosphorescent nanoparticle ("NP"), a fluorescent latex particle or microbead, a fluorescent dye molecule, such as fluorescein, carboxyfluorescein and a fluorescein derivative, or a rhodamine or its derivatives.

9. The method of Claim 1 or 2 in which said fluorophore is a fluorescence resonance energy transfer ("FRET") complex such as an intrachain or a competitive FRET-aptamer.

10. The method of Claim 1 or 2, wherein said assaying step is a sandwich assay to detect and quantify said target analyte in said bulk solution.

11. The method of Claim 1 or 2, wherein said target analyte is a whole cell, such as a bacterium, parasite, leukocyte, or cancer cell.

12. The method of Claim 1 or 2, wherein said target analyte is a protein, viral capsid protein, viral polymerase, biotoxin such as bacterial toxin, such as botulinum, cholera, tetnus, staphylococcal enterotoxin, shigatoxins or verotoxins, algal toxin, such as brevetoxin, ciguatoxin, cyanotoxin, or saxitoxin, snake or spider venom, clinically relevant protein or portions of protein (peptides) such as bone marker (e.g., collagen breakdown peptides such as CTx, NTx, OCF, Cathepsin K or its precursor ProCathepsin K, deoxypyridinoline, pyridinoline, lysylpyridinoline, or hydroxylysylpyridinoline) cytokines and interleukins, markers of myocardial infarctions (troponin, myoglobin, etc.), kidney disease, antibodies, autoimmune disorders, arthritis, or other clinically relevant macromolecules such as lipopolysaccharides (LPS, endotoxins).

13. The method of Claim 1 or 2, wherein said target analyte includes small molecules (molecules of less than 1,000 Daltons) with at least two distinct epitopes from a group including the following: pesticides, natural and synthetic amino acids and their derivatives, hydroxylysine, hydroxyproline, histidine, histamine, homocysteine, DOPA, melatonin, nitrotyrosine, short chain proteolysis products, cadaverine, putrescine, polyamines, spermine, spermidine, deoxypyridinoline, pyridinoline, lysylpyridinoline, or hydroxylysylpyridinoline, nitrogen bases of DNA or RNA, nucleosides, nucleotides, nucleotide cyclical isoforms, cAMP, cGMP, cellular metabolites, urea, uric acid, pharmaceuticals, therapeutic drugs, vitamins, illegal drugs, narcotics, hallucinogens, gamma-hydroxybutyrate (GHB), cellular mediators, cytokines, chemokines, immune modulators, neural modulators, neurotransmitters such as acetylcholine, inflammatory modulators, prostaglandins, prostaglandin metabolites, nitoaromatic and nitramine explosives, explosive breakdown products (e.g., DNT) or byproducts, quorum sensing molecules such as AHLs, steroids, hormones, and their derivatives.

14. The method of Claim 1 or 2 in which said assaying step of a target analyte is performed using one of: fluorescence intensity, time-resolved fluorescence, chemiluminescence, electrical detection, electrochemical detection, electrochemiluminescence, phosphorescence, or radioisotopic detection.

## Patentansprüche

1. Verfahren zum Sandwichdosieren, durchgeführt durch Herstellung und Verbindung von Konjugaten aus DNA
- Aptamer oder RNS - Magnetkügelchen für den Einfang und für den Nachweis eines Zielanalyten in einer Lösung, umfassend :
- das Kombinieren der besagten Lösung, mit einem Konjugat aus Aptamer und Magnetkügelchen ("Aptamer BM") und einem Konjugat aus Aptamer und Fluorophor in einer Küvette, bei dem das besagte Aptamer BM sich mit dem besagten Zielanalyten in einer ersten Bindungsstelle verbinden kann, und das besagte Konjugat aus Aptamer und Fluorophor sich mit dem besagten Zielanalyten in einer zweiten Bindungsstelle verbinden kann, um einen Analyt - Aptamer - Fluophor-Komplex zu bilden, und bei dem die besagte Küvette mit einer durchscheinende Fläche versehen ist, um ein Dosieren der Fluoreszenz zu ermöglichen,
- die Möglichkeit die Verbindung zwischen dem Aptamer-BM und dem besagten Zielanalyten in der ersten Verbindungsstelle und des besagten Konjugats aus Aptamer und Fluorophor mit dem besagten Zielanalyten in der zweiten Verbindungsstelle, um den besagten Analyt-Aptamer-Fluorophor-Komplex zu bilden, **dadurch gekennzeichnet, dass** das Verfahren ferner folgende Schritte umfasst:
- die Positionierung der Küvette derart, dass die durchscheinende Fläche im Wesentlichen senkrecht orientiert ist,
- das Anhaften des besagten Aptamer - Fluorophor - Komplexes auf der durchscheinenden Fläche der besagten Küvette durch Anlegen eines äußeren magnetischen Feldes, um das magnetische Kügelchen anzuziehen,
- die Entfernung des äußeren Magnetfeldes, und
- das Dosieren des besagten auf der durchscheinenden Fläche der Küvette anhaftenden Aptamer-Fluorophor-Komplexes durch Beleuchtung der Lösung mittels einer Erregungsquelle, ausgehend von der entgegengesetzten Seite der Küvette, und Platzierung der die anhaftenden Materialien aufweisenden Seite der Küvette in der Nähe eines Fotodetektors.

2. Verfahren nach dem Anspruch 1, bei dem die besagte Lösung sauer ist.

3. Verfahren nach Anspruch 1 oder 2, bei dem das Verfahren keinen Waschvorgang umfasst.

4. Verfahren nach Anspruch 1 oder 2, bei dem der besagte Aptamer - Fluorophor - Komplex von der Lösung wirksam getrennt ist, um die Nachweisbarkeit zu verstärken.

5. Verfahren nach Anspruch 1 oder 2, bei dem die besagte Küvette aus Polystyrol, aus einem klarsichtigen Kunststoff oder aus Glas hergestellt ist.

6. Verfahren nach dem Anspruch 5, bei dem die durchscheinende Fläche der besagten Küvette, auf der der besagte Analyt - Aptamer - Fluophor-Komplex haftet, eine quadratische, rechteckige, runde oder ovale Form aufweist, oder durch einen flachen Behälter, ein Fläschchen, ein Rohr, einen Zylinder, eine Kassette oder eine Kartusche gebildet ist.

7. Verfahren nach Anspruch 1 oder 2, bei dem das besagte Aptamer - BM und das besagte Aptamer-Fluorophor sich in der besagten Lösung nicht verbinden, Basenpaare bilden oder hybridisieren.

8. Verfahren nach Anspruch 1 oder 2, bei dem das besagte Fluorophor in dem besagten Konjugat eine fluoreszente oder phosphoreszente Nanopartikel (« NP ») mit Quantenpunkt ("PQ"), eine fluoreszente Partikel oder Mikrokugel aus Latex, ein fluoreszentes Farbstoffmolekül, wie das Fluoreszein, das Carboxyfluoreszein, und ein Derivat von dem Fluoreszein, oder das Rhodanammonium oder Derivate von diesem ist.

9. Verfahren nach Anspruch 1 oder 2, bei dem das besagte Fluorophor ein Fluoreszenz - Resonanztransfer - Komplex (« FRET »), wie eine FRET Aptamer - Intrakette oder ein kompetitiver FRET ist.

10. Verfahren nach Anspruch 1 oder 2, bei dem der besagte Dosierschritt ein Sandwich Dosieren zum Nachweis und zur Quantifizierung des besagten Zielanalyten in der besagten Lösung ist.

11. Verfahren nach Anspruch 1 oder 2, bei dem der besagte Zielanalyt eine ganze Zelle, wie eine Bakterie, ein Parasit, ein Leukozyt oder eine Krebszelle ist.

12. Verfahren nach Anspruch 1 oder 2, bei dem der besagte Zielanalyt ein Protein, ein virales Kapsid-Protein, eine virale Polymerase, ein Biotoxin, wie ein bakterielles Toxin, wie das Botulinumtoxin, das Cholera- das Tetanus-, das Staphylokokken- Enterotoxin, das Shigatoxin oder die Verotoxine, ein Algentoxin, wie das Brevetoxin, das Ciguatoxin, das Zyanotoxin, oder das Saxitoxin, ein Gift einer Schlange oder einer Spinne, ein klinisch wichtiges Protein oder Teile von Proteinen (Peptide) wie ein Knochenmarker (zum Beispiel, die Kollagenabbaupeptide, wie CTx, NTx, OCF, das Cathepsin K oder dessen Zwischenstoff, das Procathepsin K, das Desoxypyridinolin, das Pyridinolin, das Lysyl - pyridinolin oder das Hydroxilysylpyridinolin), Cytokine oder Interleukine, Marker von Infarkten des Myokards (Troponin, Myoglobin, usw.), von Nierenerkrankungen, von Antikörpern, von Störungen des Immunsystems, Arthritis, oder von anderen klinisch wichtigen Makromolekülen, wie die Lipo polysaccharide (LPS, Endotoxine), ist.

13. Verfahren nach Anspruch 1 oder 2, bei dem der besagte Zielanalyt kleine Moleküle (Moleküle von weniger als 1000 Daltons) umfasst, die mindestens zwei unterschiedlichen Epitopen aufweisen, und zwar aus der Gruppe umfassend: Pestiziden, natürliche und synthetische Aminosäuren und deren Derivate, das Hydroxylysin, das Hydroxiprolin, das Histidin, das Histamin, das Homozystein, das Dopa, das Melatonin, das Nitrotyrosin, Proteolyse-Produkte mit kurzer Kette, des chimiokines, des modulateurs immunitaires, des modulateurs neuronaux, das Kadaverin, das Putreszin, Polyamine, das Spermin, das Spermidin, das Desoxypyridinolin, das Pyridinolin, das Lysyl pyridinolin oder das Hydroxilysyl pyridinolin, die Stickstoffbasen der DNA oder der RNS, Nukleoside, Nukleotide, zyklische Isoformen von Nukleotiden, cAMP, cGMP, Zellenmetabolite, Urea, Harnsäure, pharmazeutische Agense, therapeutische Medikamente, Vitaminen, illegale Drogen, Narkotika, Halluzinogene, Gamma hydroxybutyrate (GHB), Zellmediatoren, Zytokine, Chemokine, Immunitätsmodultoren, Neuromodulatoren, Neurotransmitter, wie das Azethylcholin, Entzündungsmodulatoren, Prostaglandine, Metabolite von Prostaglandin, nitroaromatische Sprengstoffe und Nitramin, Produkte zur explosiven Zerstörung (zum Beispiel DNT) oder sekundäre Produkte, Moleküle zum Nachweis von Quorum (Quorum sensing), wie die AHL, die Steroide, die Hormonen und deren Derivate.

14. Verfahren nach Anspruch 1 oder 2, bei dem der besagte Schritt des Dosierens eines Zielanalyten unter optionaler Anwendung der Intensität der Fluoreszenz, der zeitaufgelösten Fluoreszenz, der Chemolumineszenz, des elektrischen Nachweises, des elektrochemischen Nachweises, der elektrochemischen Lumineszenz, der Phosphoreszenz oder des radio isotopischen Nachweises durchgeführt wird.

## Revendications

1. Procédé de dosage en sandwiche, effectué par production et assemblage de conjugués aptamère ADN ou ARN-bille magnétique, pour la capture et la détection d'un analyte cible dans une solution, comprenant :
la combinaison de ladite solution, d'un conjugué aptamère-bille magnétique (« aptamère-BM ») et d'un conjugué aptamère-fluorophore dans une cuvette, où ledit aptamère-BM est capable de se lier audit analyte cible en un premier site de liaison et ledit conjugué aptamère-fluorophore est capable de se lier audit analyte cible en un deuxième site de liaison pour former un complexe analyte-aptamère-fluorophore, et où ladite cuvette présente une surface translucide de manière à permettre un dosage de la fluorescence ;
laisser ledit aptamère-BM se lier audit analyte cible en un premier site de liaison et audit conjugué aptamère-fluorophore se lier audit analyte cible en un deuxième site de liaison pour former ledit complexe analyte-aptamère-fluorophore ;
**caractérisé en ce que** le procédé comprend en outre, le positionnement de la cuvette de telle sorte que la surface translucide soit orientée de manière sensiblement verticale ;
l'adhésion dudit complexe analyte-aptamère-fluorophore à la surface translucide de ladite cuvette par application d'un champ magnétique externe afin d'attirer ladite bille magnétique ;
l'enlèvement du champ magnétique externe, et
le dosage dudit complexe analyte-aptamère-fluorophore qui adhère à la surface translucide de ladite cuvette par illumination de la solution depuis la face opposée de la cuvette à l'aide d'une source d'excitation et placement du côté de la cuvette présentant les matériaux adhérant près d'un photodétecteur.

2. Procédé selon la revendication 1, où ladite solution est acide.

3. Procédé selon la revendication 1 ou 2, où ledit procédé ne comprend pas d'étape de lavage.

4. Procédé selon la revendication 1 ou 2, où ledit complexe analyte-aptamère-fluorophore est efficacement séparé de la solution pour amplifier la détectabilité.

5. Procédé selon la revendication 1 ou 2, où ladite cuvette est constituée de polystyrène, d'une matière plastique transparente ou de verre.

6. Procédé selon la revendication 5, où la surface translucide de ladite cuvette, sur laquelle ledit complexe analyte-aptamère-fluorophore adhère, a une forme carrée, rectangulaire, ronde, ovale ou est formée d'un récipient, flacon, tube, cylindre, cassette ou cartouche plane.

7. Procédé selon la revendication 1 ou 2, où ledit aptamère-BM et ledit aptamère-fluorophore ne vont pas se lier, former des paires de bases ou s'hybrider l'un à l'autre dans ladite solution.

8. Procédé selon la revendication 1 ou 2, où ledit fluorophore dans ledit conjugué aptamère-fluorophore est une nanoparticule (« NP ») à point quantique (« PQ »), fluorescente ou phosphorescente, une particule ou microbille fluorescente en latex, une molécule de colorant fluorescent comme la fluorescéine, la carboxyfluorescéine et un dérivé de la fluorescéine, ou la rhodamine ou ses dérivés.

9. Procédé selon la revendication 1 ou 2, où ledit fluorophore est un complexe de transfert d'énergie de fluorescence par résonance (« FRET ») comme un FRET-aptamère intrachaine ou compétitif.

10. Procédé selon la revendication 1 ou 2, où ladite étape de dosage est un dosage en sandwiche destiné à détecter et quantifier ledit analyte cible dans la solution.

11. Procédé selon la revendication 1 ou 2, où ledit analyte cible est une cellule entière, comme une bactérie, un parasite, un leucocyte ou une cellule cancéreuse.

12. Procédé selon la revendication 1 ou 2, où ledit analyte cible est une protéine, une protéine de capside viral, une polymérase virale, une biotoxine comme une toxine bactérienne telle que la botuline, l'entérotoxine du choléra, du tétanos, de staphylocoque, des shigatoxines ou des vérotoxines, une toxine d'algue telle que la brévétoxine, la ciguatoxine, la cyanotoxine ou la saxitoxine, du venin de serpent ou d'araignée, une protéine cliniquement importante ou des parties de protéine (peptides) comme un marqueur des os (par exemple, peptides de destruction du collagène tels que CTx, NTx, OCF, cathepsine K ou son précurseur, procathepsine K, désoxypyridinoline, pyridinoline, lysylpyridinoline ou hydroxylysylpyridinoline), des cytokines et interleukines, des marqueurs d'infarctus du myocarde (troponine, myoglobine, etc.), de maladies rénales, des anticorps, des désordres auto-immuns, l'arthrite ou d'autres macromolécules cliniquement importantes telles que des lipopolysaccharides (LPS, endotoxines).

13. Procédé selon la revendication 1 ou 2, où ledit analyte cible comprend de petites molécules (molécules de moins de 1000 Daltons) ayant au moins deux épitopes distincts, parmi le groupe comprenant des pesticides, des acides aminés naturels et synthétiques et leurs dérivés, l'hydroxylysine, l'hydroxyproline, l'histidine, l'histamine, l'homocystéine, le DOPA, la mélatonine, la nitrotyrosine, des produits de protéolyse à chaine courte, la cadavérine, la putrescine, des polyamines, la spermine, la spermidine, la désoxypyridinoline, la pyridinoline, la lysylpyridinoline, ou l'hydroxylysylpyridinoline, des bases azotées de l'ADN ou de l'ARN, des nucléosides, des nucléotides, des isoformes cycliques de nucléotide, cAMP, cGMP, des métabolites cellulaires, l'urée, l'acide urique, des agents pharmaceutiques, des médicaments thérapeutiques, des vitamines, des drogues illégales, des narcotiques, des hallucinogènes, le gamma-hydroxybutyrate (GHB), des médiateurs cellulaires, des cytokines, des chimiokines, des modulateurs immunitaires, des modulateurs neuronaux, des neurotransmetteurs tels que l'acétylcholine, des modulateurs de l'inflammation, des prostaglandines, des métabolites de prostaglandine, des explosifs nitroaromatiques et nitramine, des produits de destruction explosive (par exemple, DNT) ou des produits secondaires, des molécules de détection de quorum (quorum sensing) telles que les AHL, des stéroïdes, des hormones et leurs dérivés.

14. Procédé selon la revendication 1 ou 2, où ladite étape de dosage d'un analyte cible est effectuée en utilisant l'un parmi : l'intensité de fluorescence, la fluorescence en temps résolu, la chimioluminescence, la détection électrique, la détection électrochimique, l'électrochimioluminescence, la phosphorescence ou la détection radioisotopique.
